(19) Europäisches Patentamt
European Patent Office
Office européen des brevets

(11) **EP 4 089 621 A1**

(12) **EUROPEAN PATENT APPLICATION**
published in accordance with Art. 153(4) EPC

(43) Date of publication:
**16.11.2022 Bulletin 2022/46**

(21) Application number: **20912364.5**

(22) Date of filing: **25.12.2020**

(51) International Patent Classification (IPC):
**G06Q 50/12** $^{(2012.01)}$    **G01N 33/02** $^{(2006.01)}$

(52) Cooperative Patent Classification (CPC):
**G01N 33/02; G06Q 50/12**

(86) International application number:
**PCT/JP2020/048731**

(87) International publication number:
**WO 2021/140961 (15.07.2021 Gazette 2021/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.01.2020 JP 2020002565**

(71) Applicant: **Sony Group Corporation**
**Tokyo 108-0075 (JP)**

(72) Inventors:
• **NASHIDA Tatsushi**
**Tokyo 108-0075 (JP)**
• **SPRANGER Michael Siegfried**
**Tokyo 108-0075 (JP)**
• **FUJITA Masahiro**
**Tokyo 108-0075 (JP)**

(74) Representative: **D Young & Co LLP**
**120 Holborn**
**London EC1N 2DY (GB)**

(54) **INFORMATION PROCESSING DEVICE AND INFORMATION PROCESSING METHOD**

(57)    The present technology relates to an information processing device and an information processing method that achieve appropriate presentation of the affinity of ingredients in combination.

An information processing device according to an aspect of the present technology presents, with use of sensor information obtained by measuring, by a sensor, a flavor of an ingredient that is used in cooking, ingredient affinity information indicating an affinity of the ingredients in combination, and flavor subjective information indicating a subjective evaluation by people regarding the flavor of the ingredient or a flavor of the ingredients in combination, the affinity of the ingredients in combination. The present technology is applicable to a computer that is provided in a kitchen.

FIG.2

EP 4 089 621 A1

**Description**

[Technical Field]

The present technology relates in particular to an information processing device and an information processing method that achieve appropriate presentation of the affinity of ingredients in combination.

[Background Art]

In recent years, attention has been paid to a technology for analyzing the flavors of food or drink by a sensor to scientifically determine the affinity of the food or drink in combination.

PTL 1 discloses a technology for suggesting an image corresponding to an intermediate ingredient obtained by preparation for cooking, in association with information indicating the process included in the preparation.

PTL 2 discloses a technology for preparing information regarding the nutrient efficacy of each combination of symptoms and nutrients and information regarding the nutrient content of each combination of ingredients and nutrients and suggesting ingredients suitable for a symptom input by the user, according to the nutrient efficacy and content.

[Citation List]

[Patent Literature]

[PTL 1]
Japanese Patent Laid-open No. 2019-20912
[PTL 2]
PCT Patent Publication No. WO2017/085777

[Summary]

[Technical Problem]

In the technologies described above, ingredient information is just simply presented, and a method for presenting, to a cook, ingredient information in conjunction with the action of the cook during cooking is not disclosed.

It is convenient if new ingredient combinations can be presented with reference to an ingredient selected by a cook, in conjunction with the action of the cook.

The present technology has been made in view of such a circumstance and makes it possible to appropriately present the affinity of ingredients in combination.

[Solution to Problem]

According to an aspect of the present technology, there is provided an information processing device including a presentation unit configured to present, using sensor information obtained by measuring, by a sensor, a flavor of an ingredient that is used in cooking, ingredient affinity information indicating an affinity of the ingredients in combination, and flavor subjective information indicating a subjective evaluation by people regarding the flavor of the ingredient or a flavor of the ingredients in combination, the affinity of the ingredients in combination.

According to an aspect of the present technology, with use of sensor information obtained by measuring, by a sensor, a flavor of an ingredient that is used in cooking, ingredient affinity information indicating an affinity of the ingredients in combination, and flavor subjective information indicating a subjective evaluation by people regarding the flavor of the ingredient or a flavor of the ingredients in combination, the affinity of the ingredients in combination is presented.

[Brief Description of Drawings]

[FIG. 1]

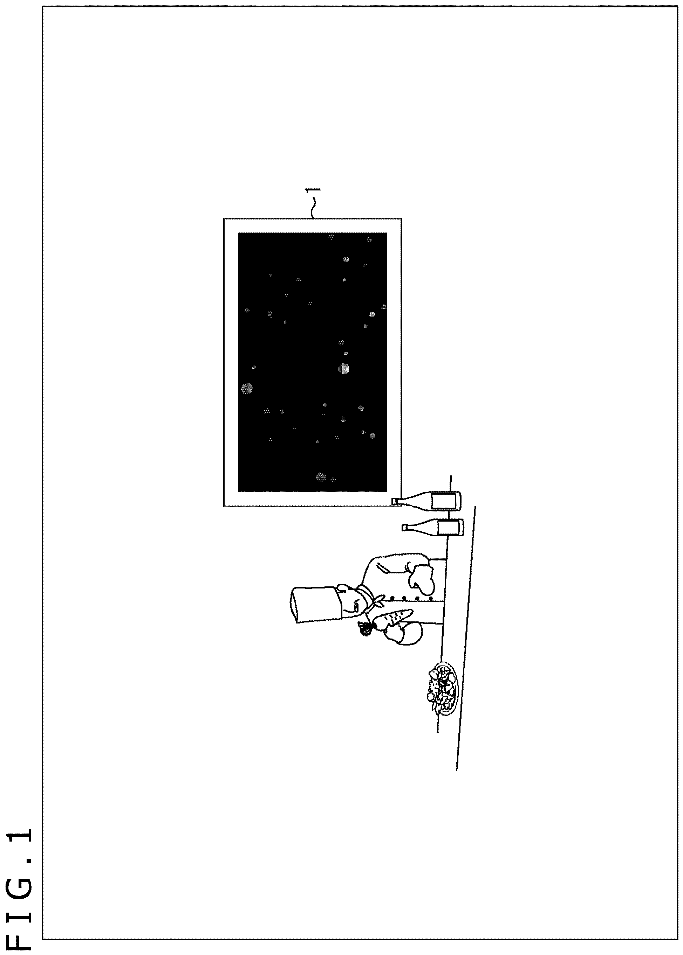

FIG. 1 is a diagram illustrating a cooking simulation device according to an embodiment of the present technology.
[FIG. 2]

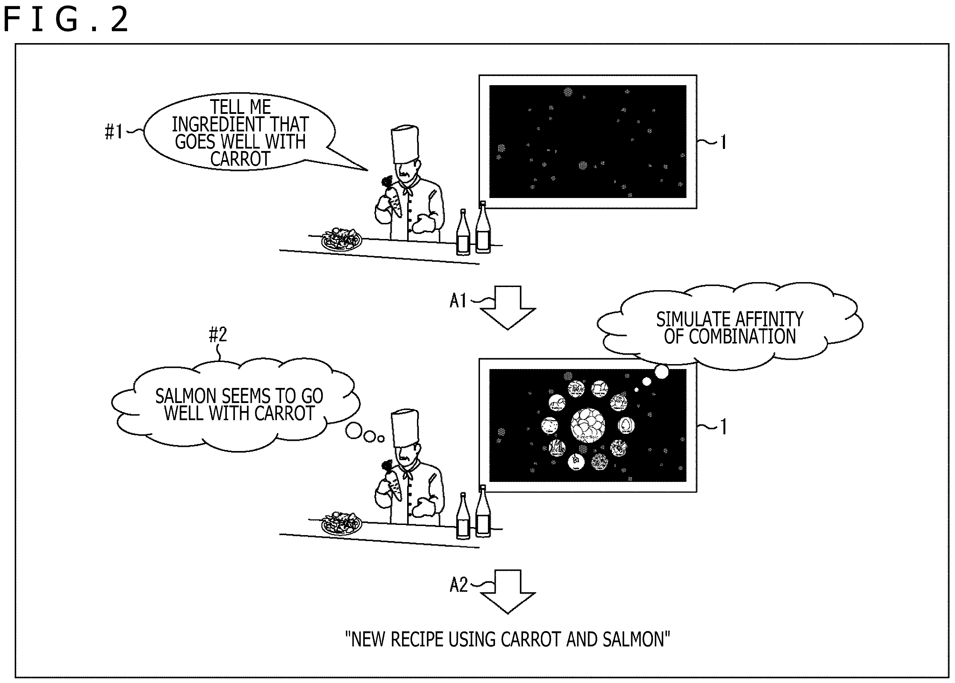

FIG. 2 is a diagram illustrating an exemplary simulation result presentation.

[FIG. 3]

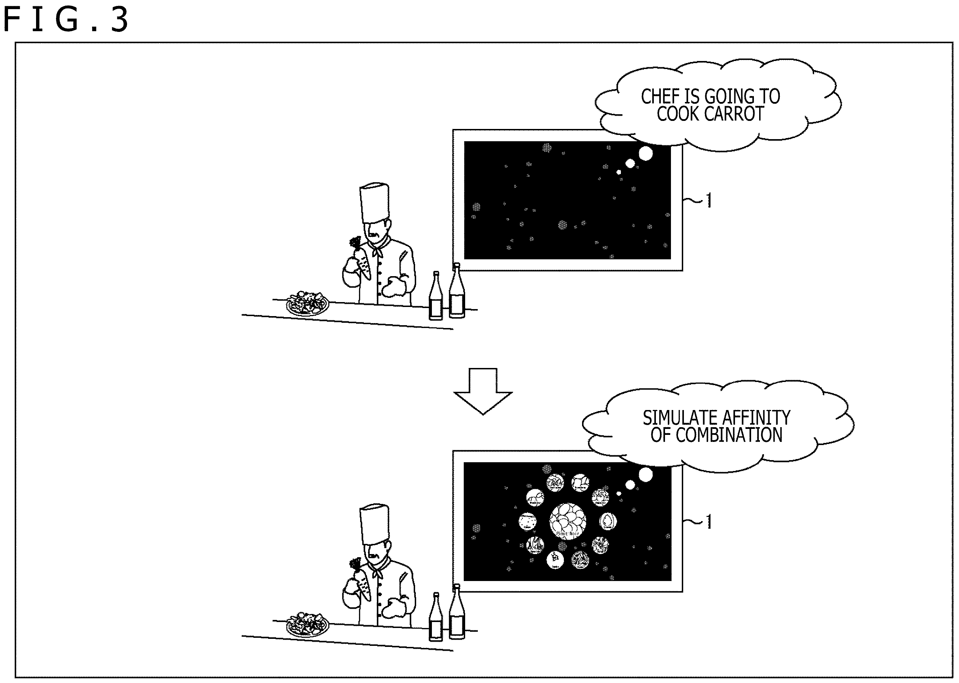

FIG. 3 is a diagram illustrating another exemplary simulation result presentation.

[FIG. 4]

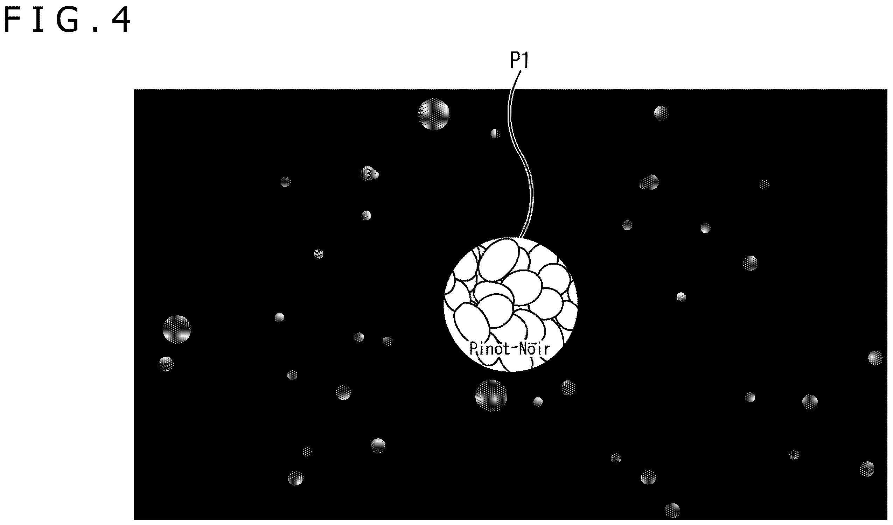

FIG. 4 is a diagram illustrating an exemplary simulation screen.

[FIG. 5]

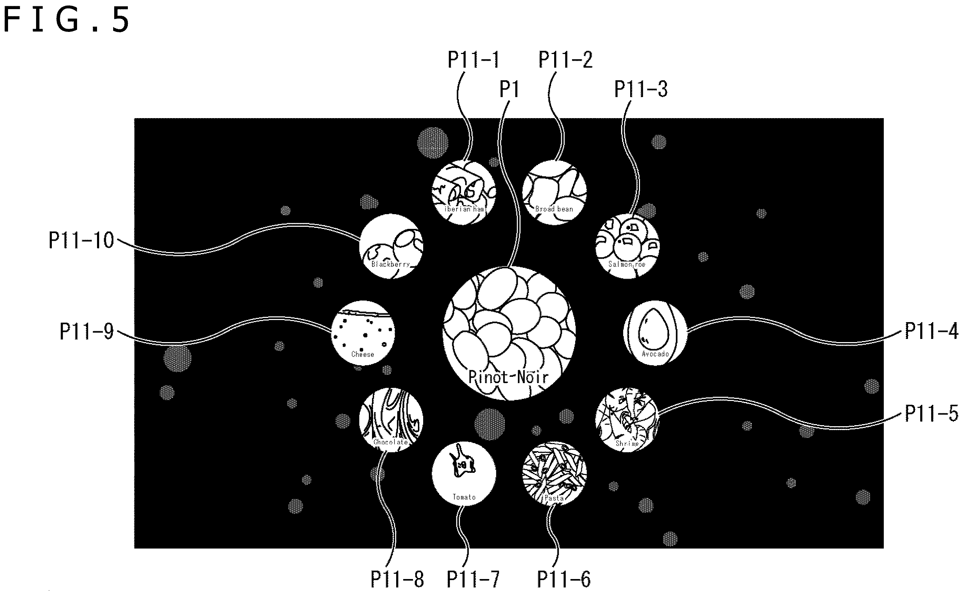

FIG. 5 is a diagram illustrating an exemplary simulation screen.

[FIG. 6]

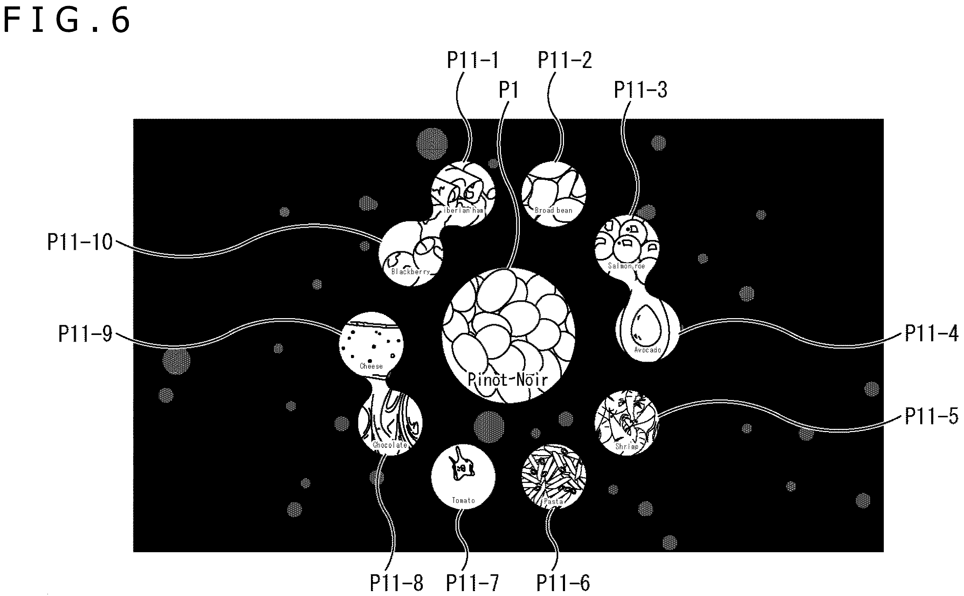

FIG. 6 is a diagram illustrating an exemplary simulation screen.

[FIG. 7]

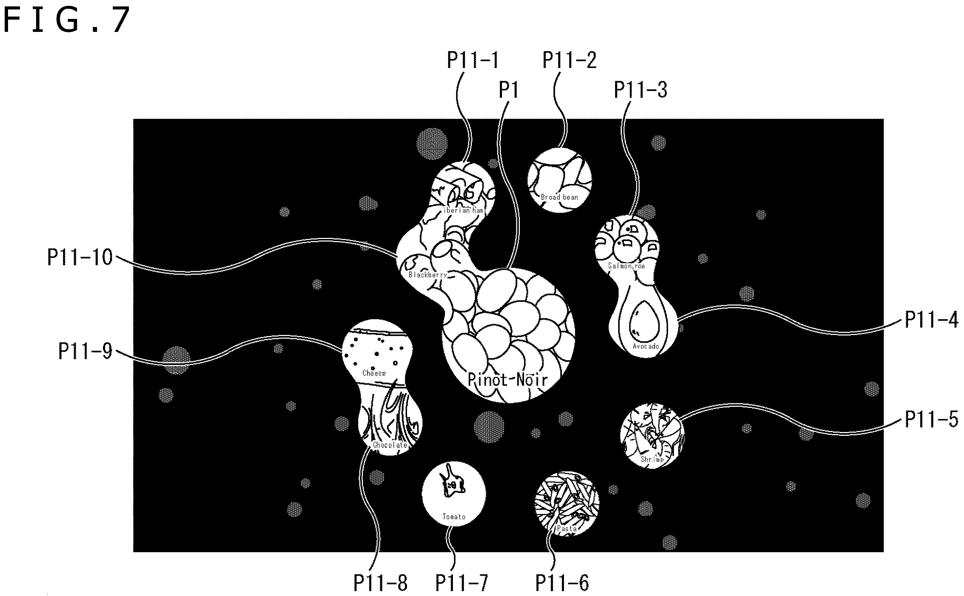

FIG. 7 is a diagram illustrating an exemplary simulation screen.

[FIG. 8]

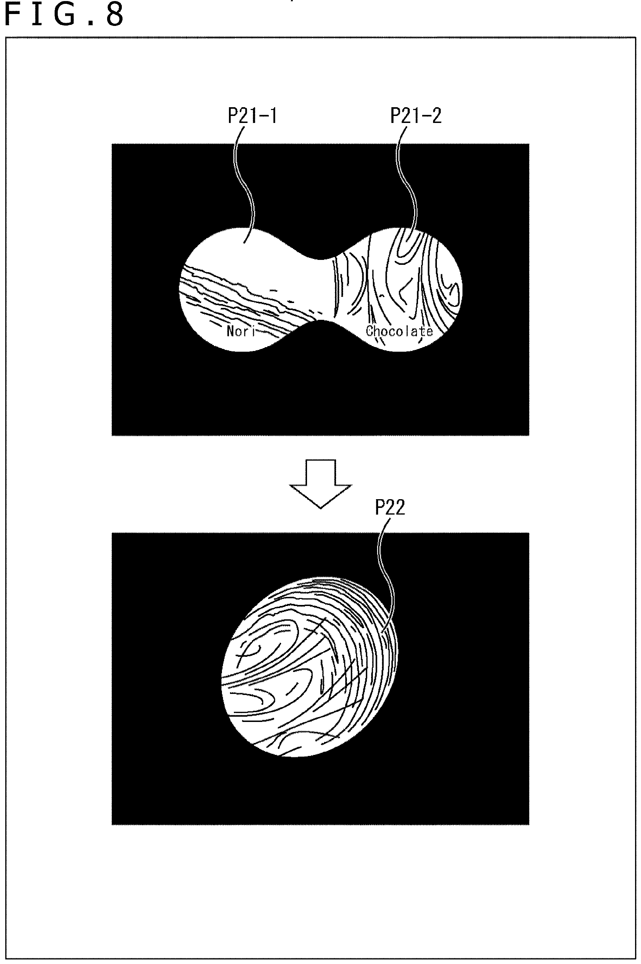

FIG. 8 is a diagram illustrating icons being merged, in an enlarged manner.

[FIG. 9]

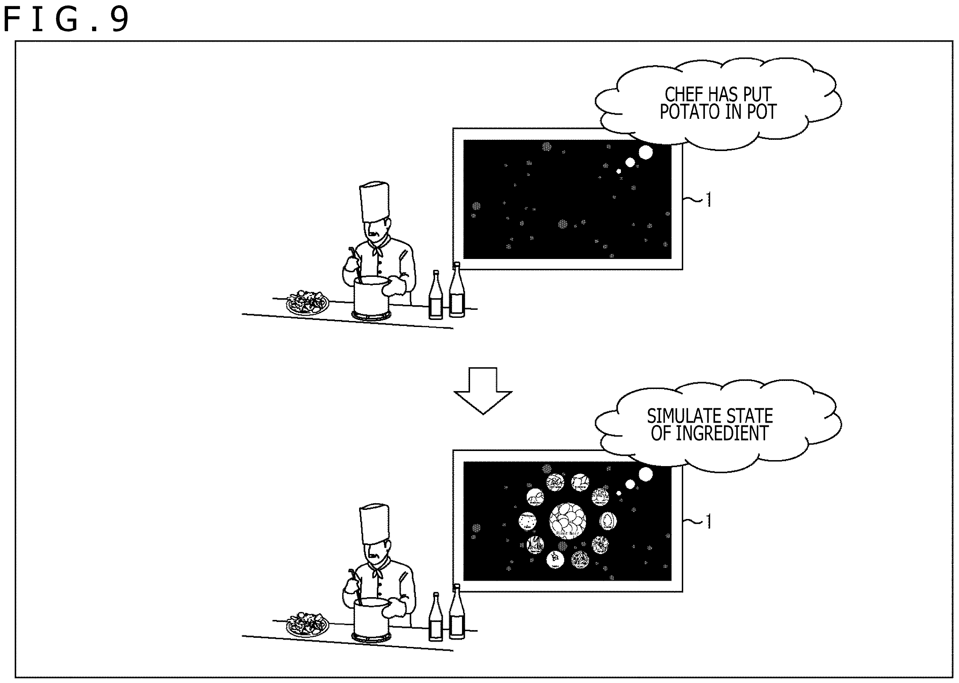

FIG. 9 is a diagram illustrating an exemplary simulation result presentation.

[FIG. 10]

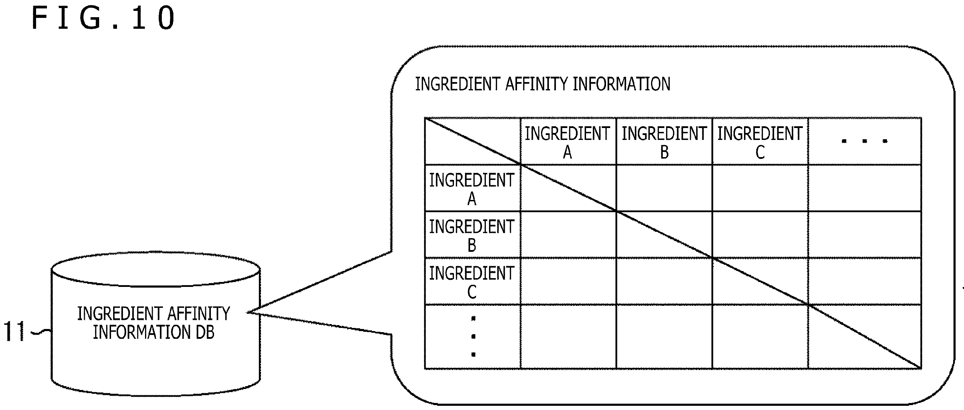

FIG. 10 is a diagram illustrating an example of an ingredient affinity information DB.

[FIG. 11]

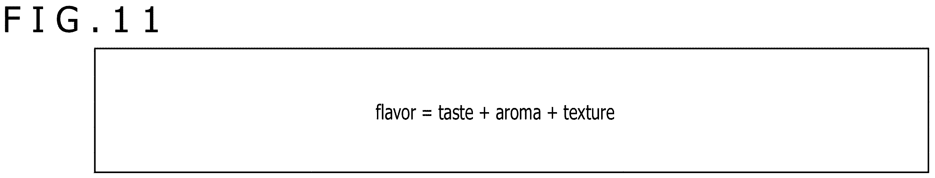

FIG. 11 is a diagram illustrating exemplary components of flavor.

[FIG. 12]

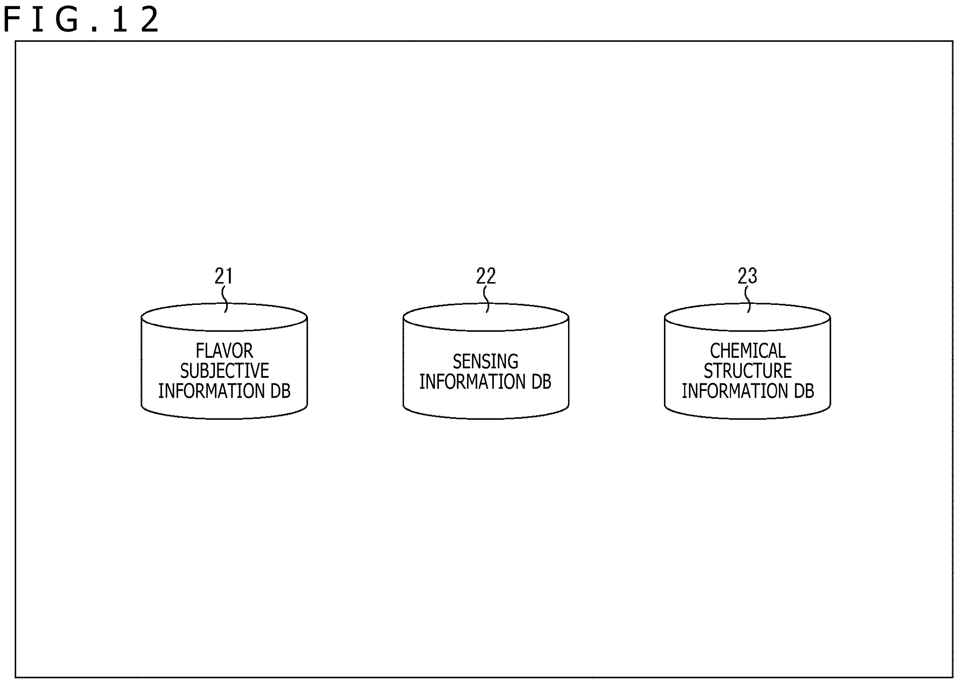

FIG. 12 is a diagram illustrating exemplary information that is used for a simulation.

[FIG. 13]

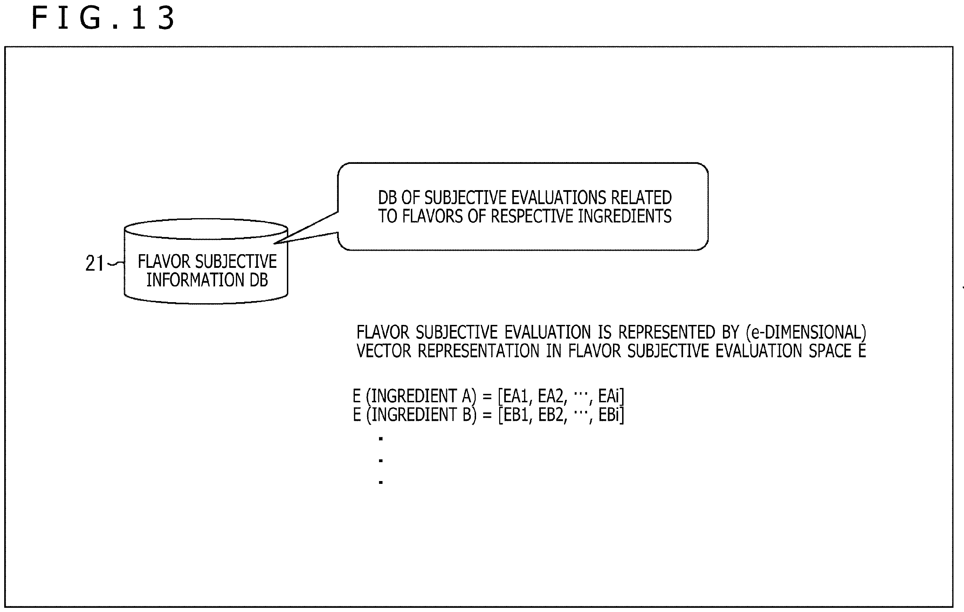

FIG. 13 is a diagram illustrating exemplary information that is stored in a flavor subjective information DB.

[FIG. 14]

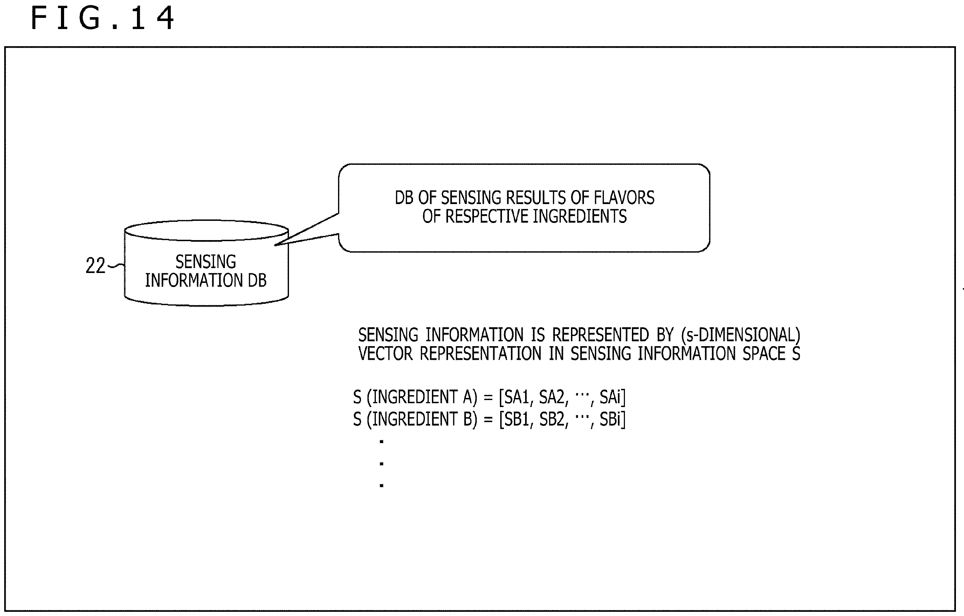

FIG. 14 is a diagram illustrating exemplary information that is stored in a sensing information DB.

[FIG. 15]

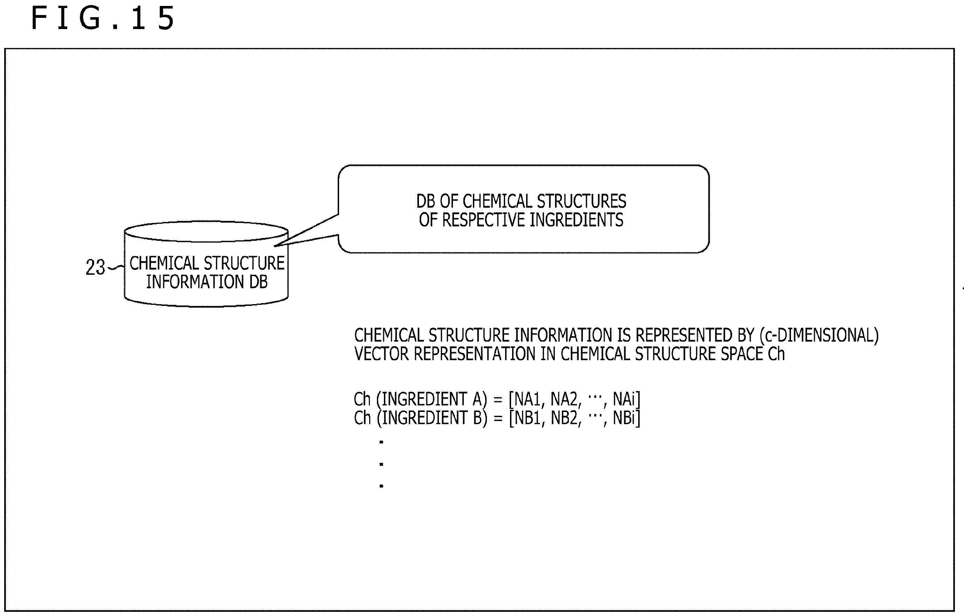

FIG. 15 is a diagram illustrating exemplary information that is stored in a chemical structure information DB.

[FIG. 16]

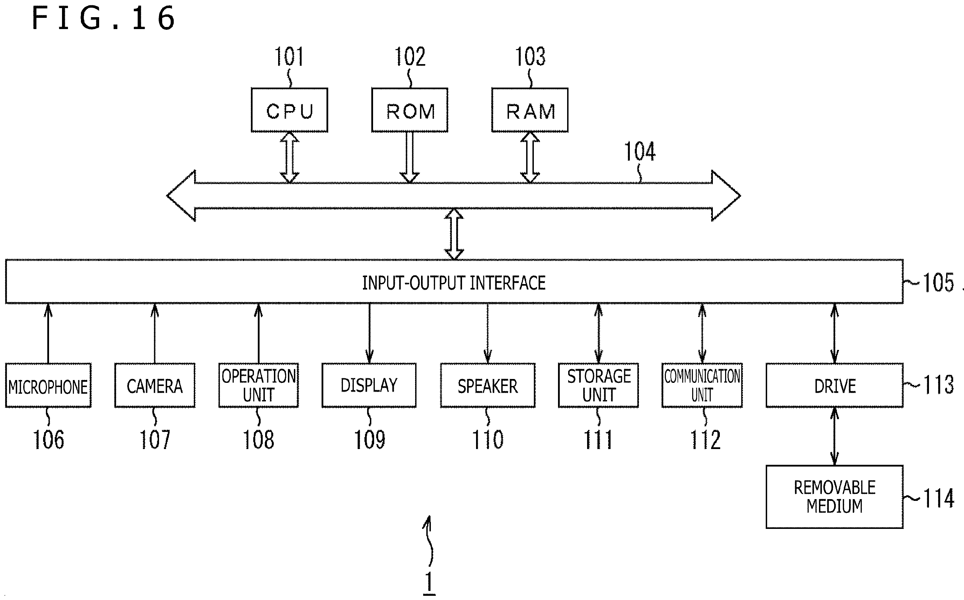

FIG. 16 is a block diagram illustrating a configuration example of the hardware of the cooking simulation device.

[FIG. 17]

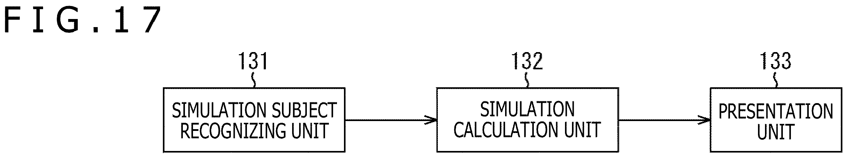

FIG. 17 is a block diagram illustrating a functional configuration example of the cooking simulation device.

[FIG. 18]

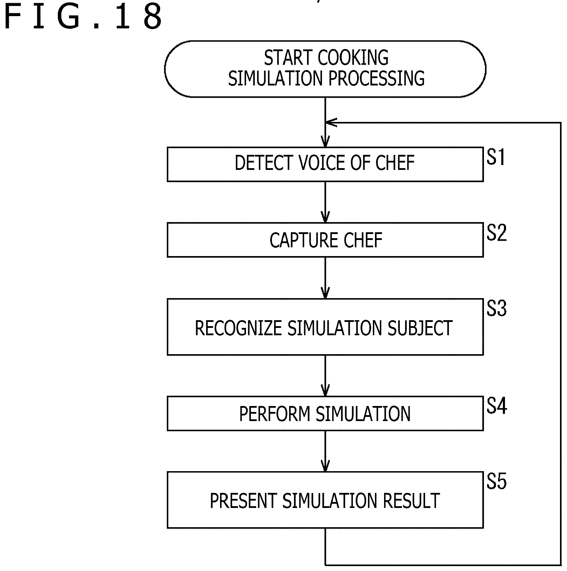

FIG. 18 is a flowchart illustrating cooking simulation processing.

[FIG. 19]

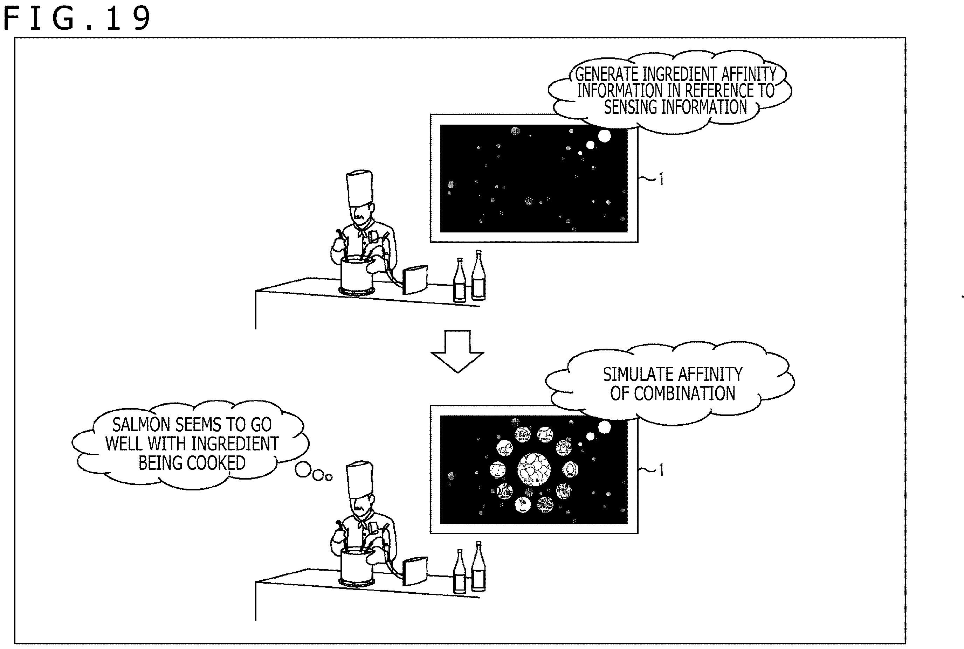

FIG. 19 is a diagram illustrating an exemplary simulation result presentation.

[FIG. 20]

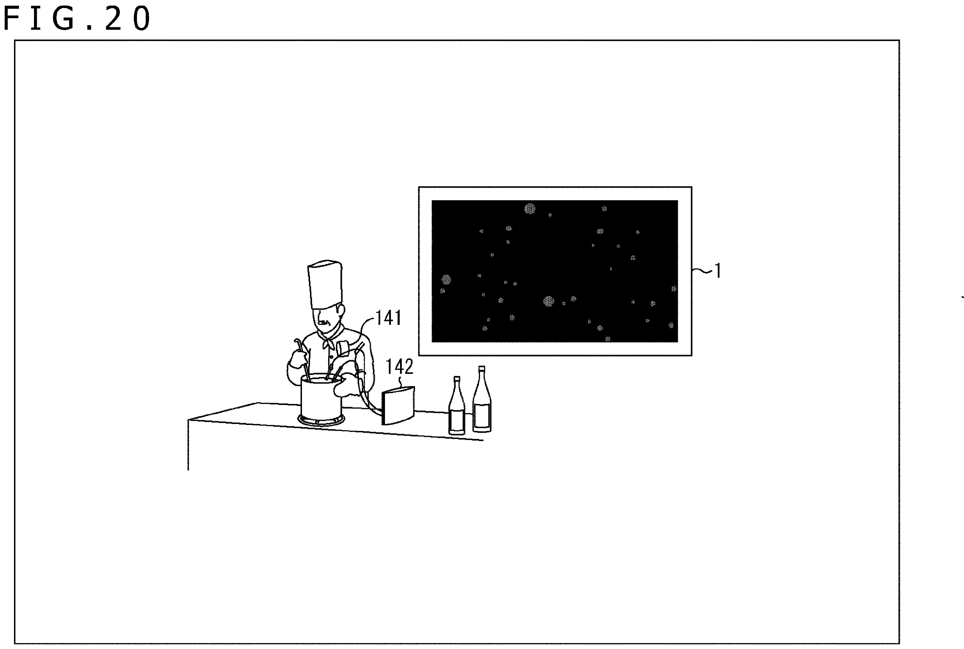

FIG. 20 is a diagram illustrating a kitchen and its surroundings in an enlarged manner.

[FIG. 21]

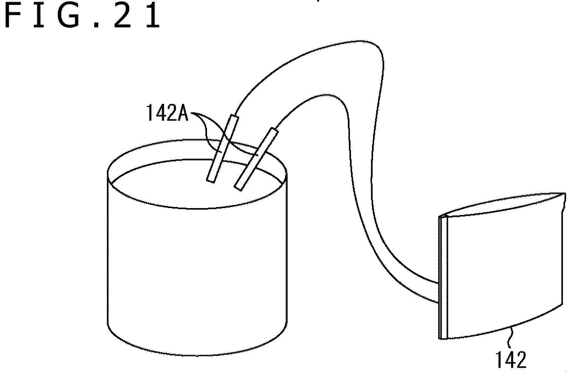

FIG. 21 is a diagram illustrating the configuration of a taste sensor.

[FIG. 22]

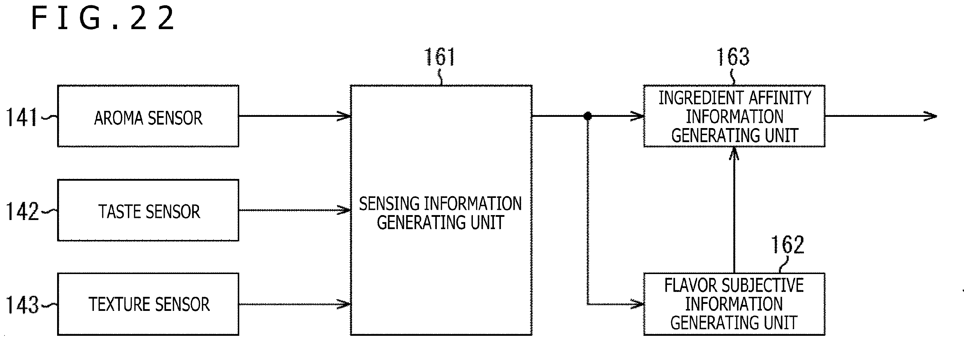

FIG. 22 is a block diagram illustrating a configuration example of the cooking simulation device.

[FIG. 23]

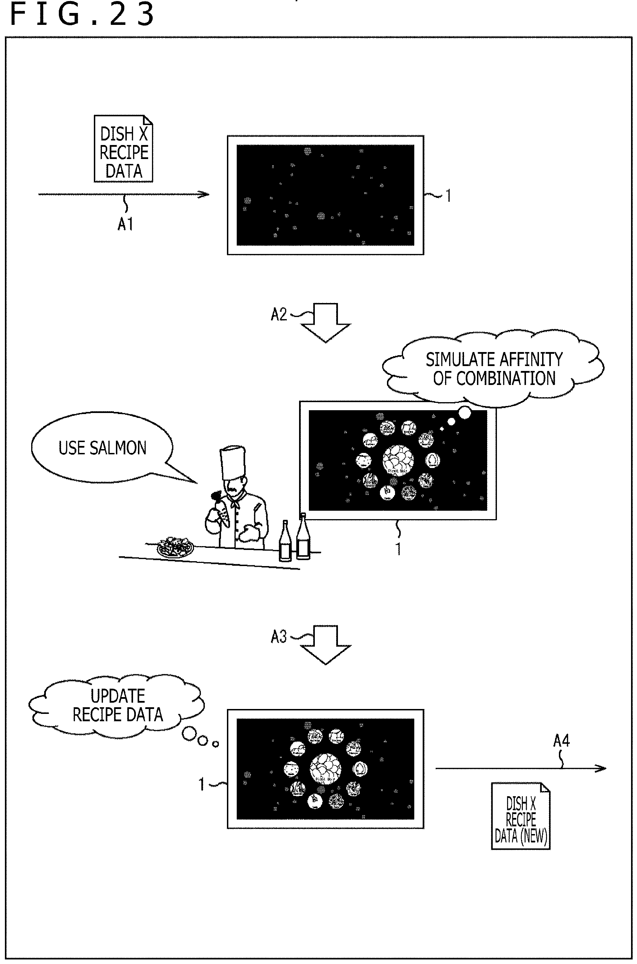

FIG. 23 is a diagram illustrating an exemplary flow of NEW recipe data generation.

[FIG. 24]

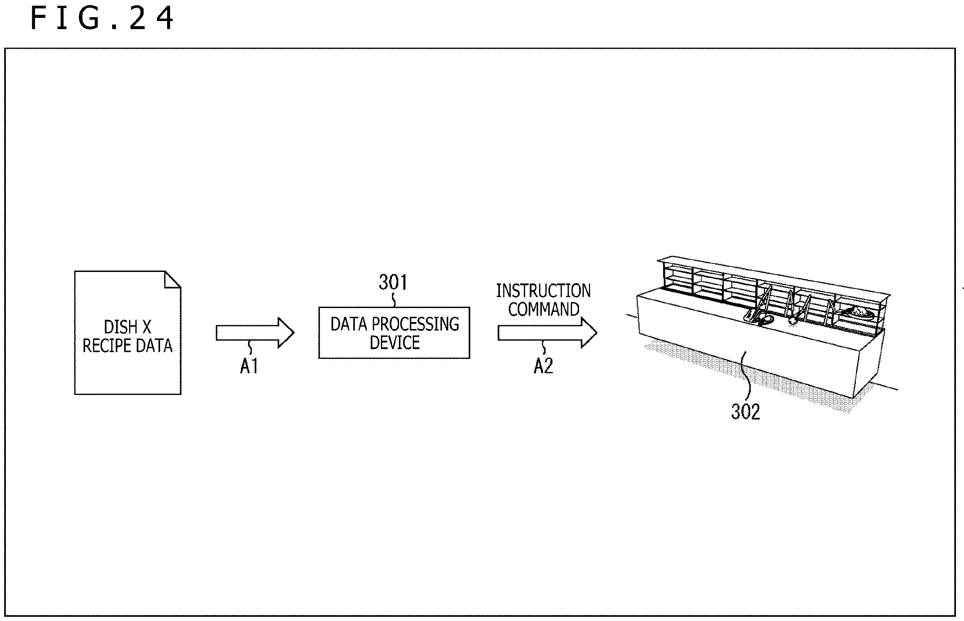

FIG. 24 is a diagram illustrating a configuration example of a control system using recipe data.

[FIG. 25]

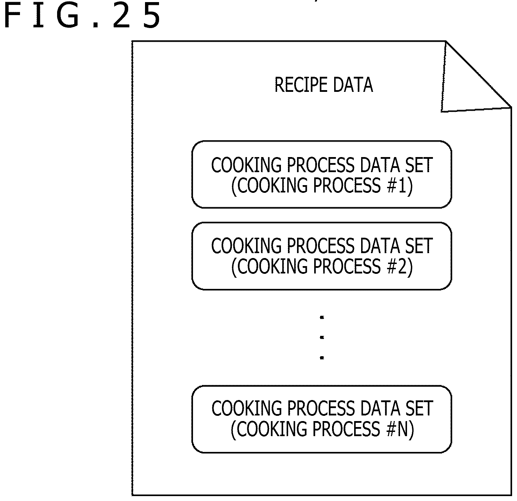

FIG. 25 is a diagram illustrating exemplary content described in recipe data.

[FIG. 26]

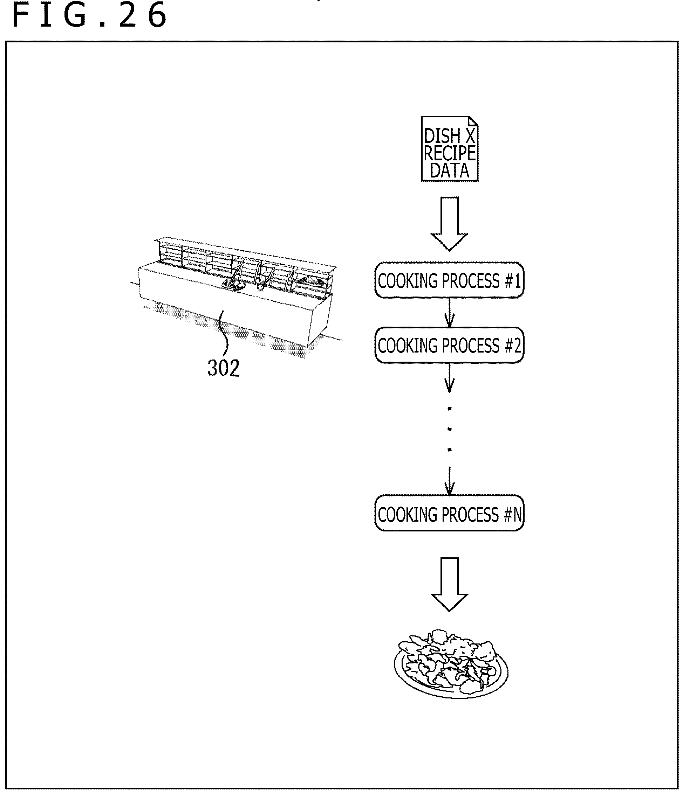

FIG. 26 is a diagram illustrating an exemplary flow of dish reproduction based on recipe data.

[FIG. 27]

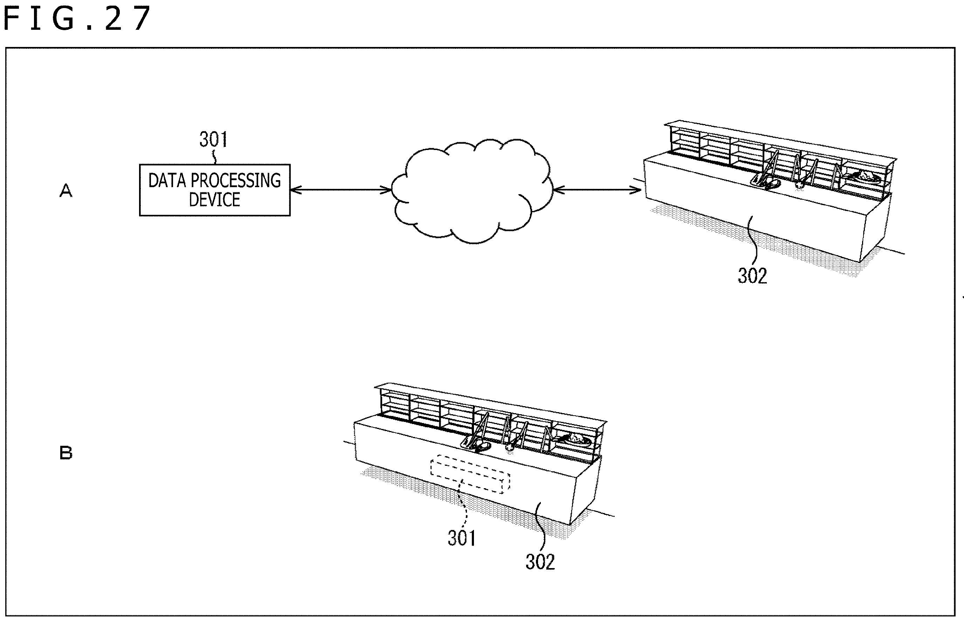

FIG. 27 depicts diagram illustrating a placement example of a data processing device.

[FIG. 28]

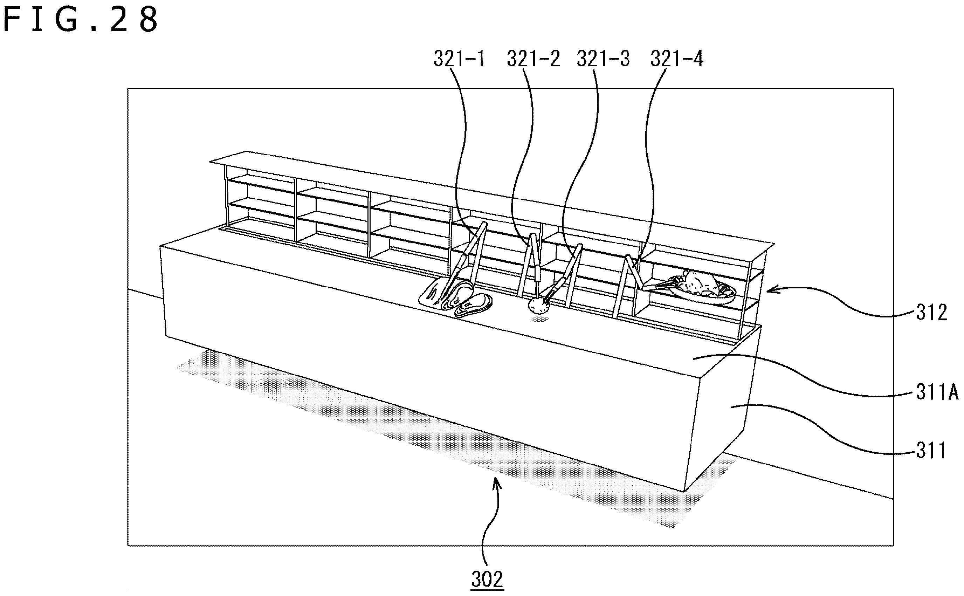

FIG. 28 is a perspective view illustrating the outer appearance of a cooking robot.

[FIG. 29]

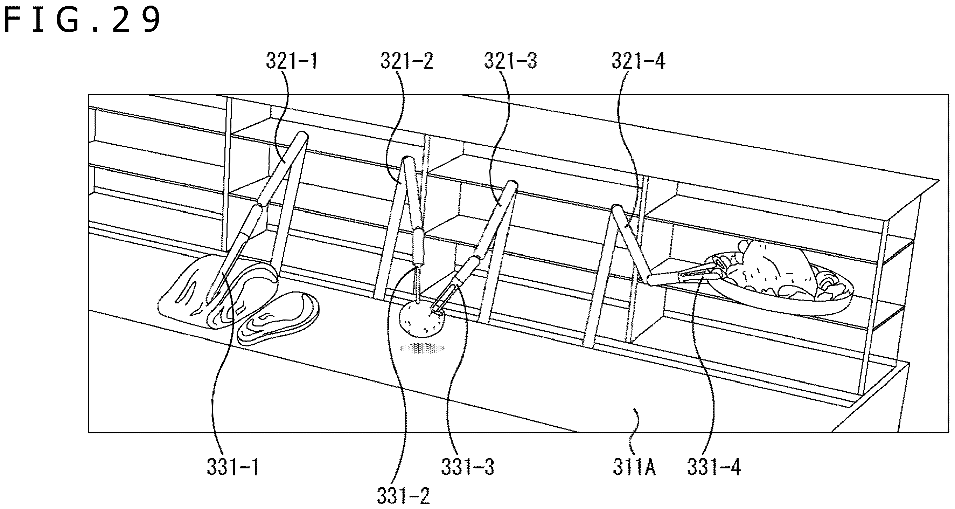

FIG. 29 is a diagram illustrating cooking arms in an enlarged manner.

[FIG. 30]

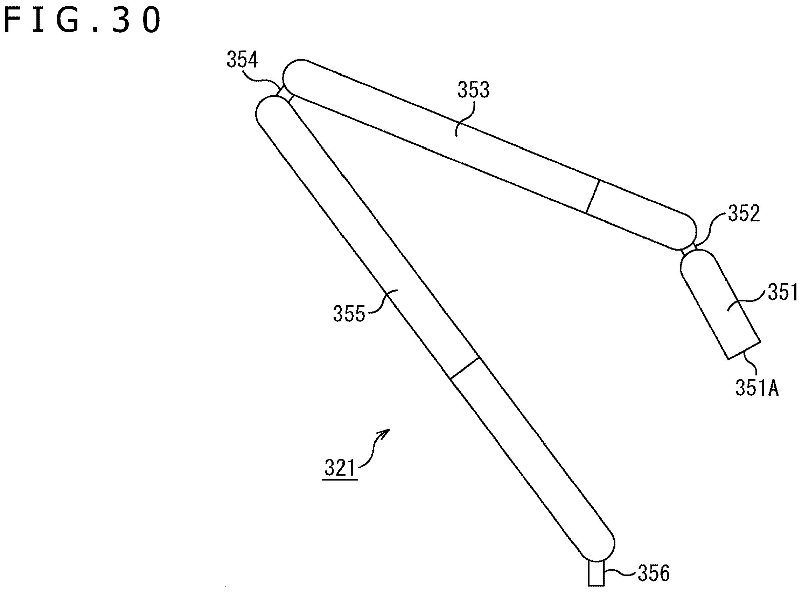

FIG. 30 is a diagram illustrating the outer appearance of the cooking arm.

[FIG. 31]

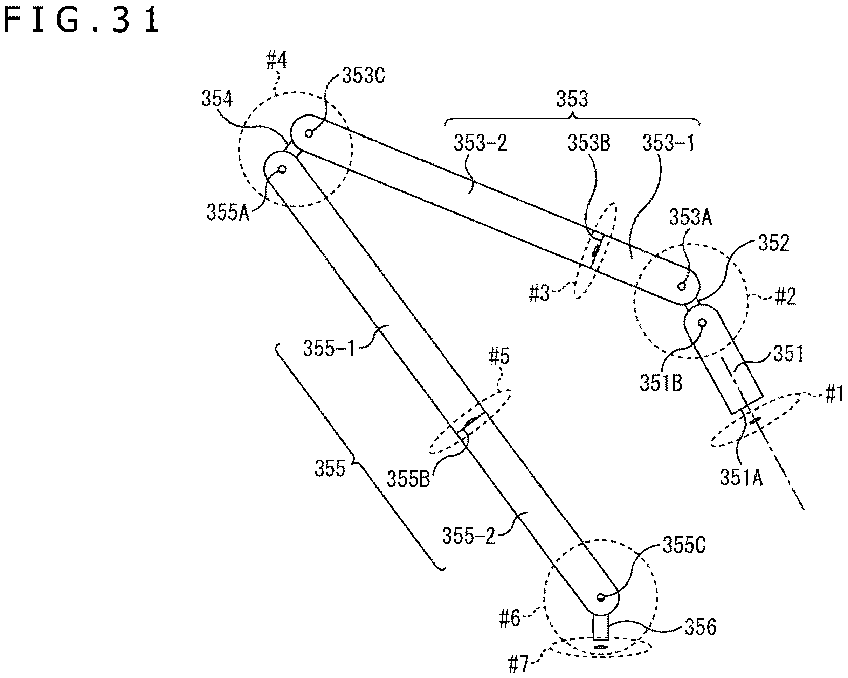

FIG. 31 is a diagram illustrating an exemplary range of motion of each portion of the cooking arm.

[FIG. 32]

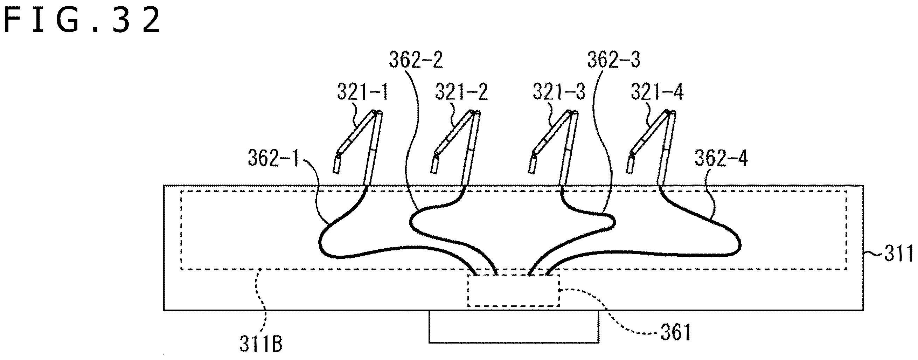

FIG. 32 is a diagram illustrating exemplary connection between the cooking arms and a controller.

[FIG. 33]

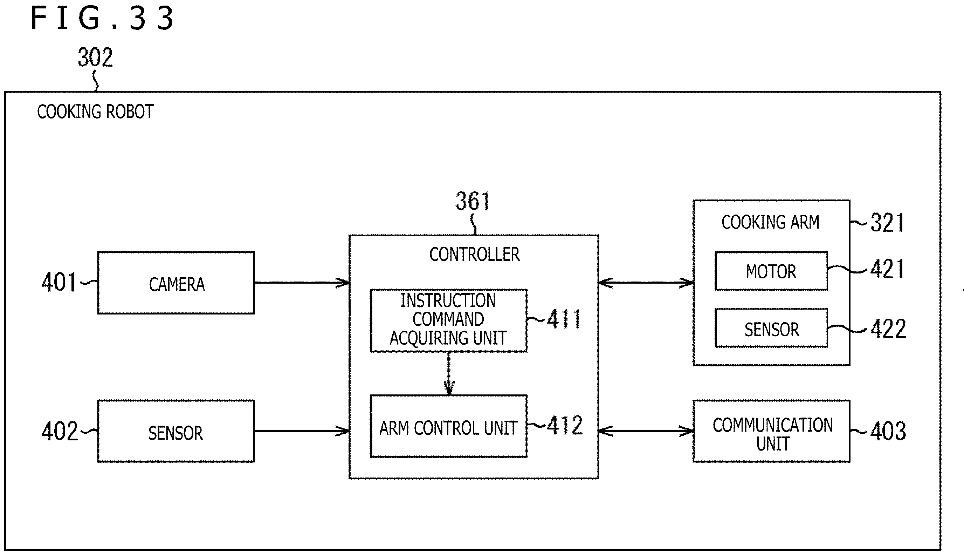

FIG. 33 is a block diagram illustrating a configuration example of the cooking robot.

[FIG. 34]

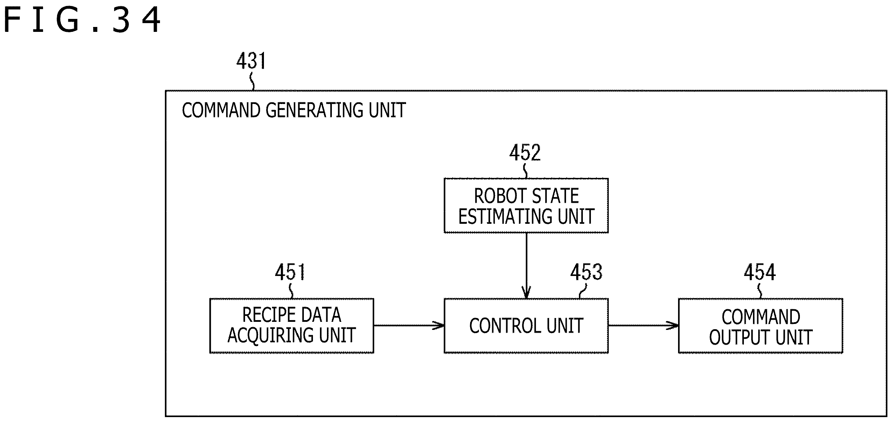

FIG. 34 is a block diagram illustrating a functional configuration example of the data processing device.

[FIG. 35]

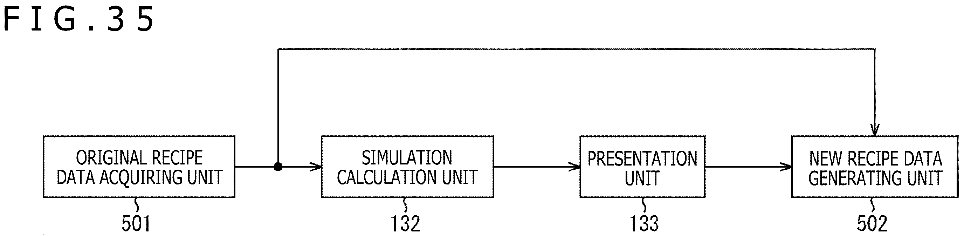

FIG. 35 is a block diagram illustrating a functional configuration example of the cooking simulation device.

[FIG. 36]

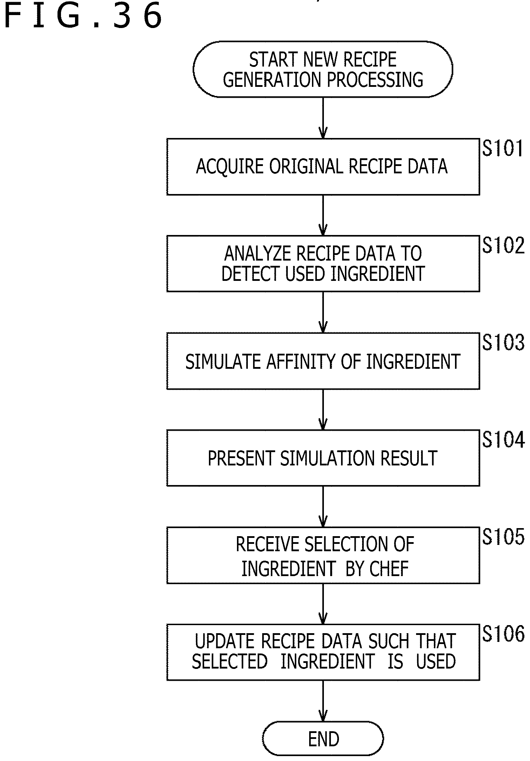

FIG. 36 is a flowchart illustrating NEW recipe data generation processing by the cooking simulation device.

[FIG. 37]

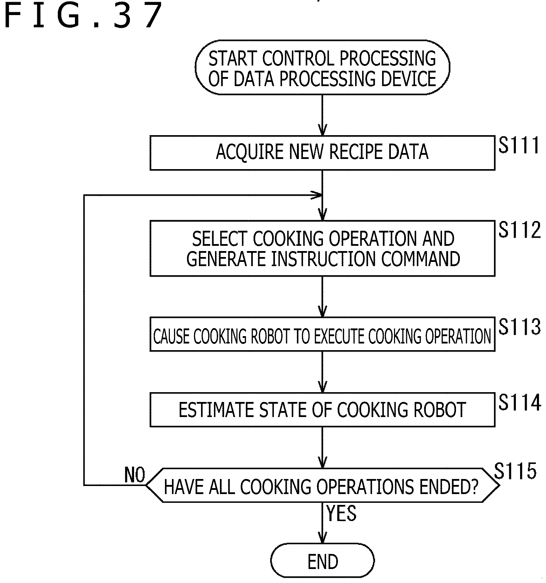

FIG. 37 is a flowchart illustrating control processing by the data processing device.

[FIG. 38]

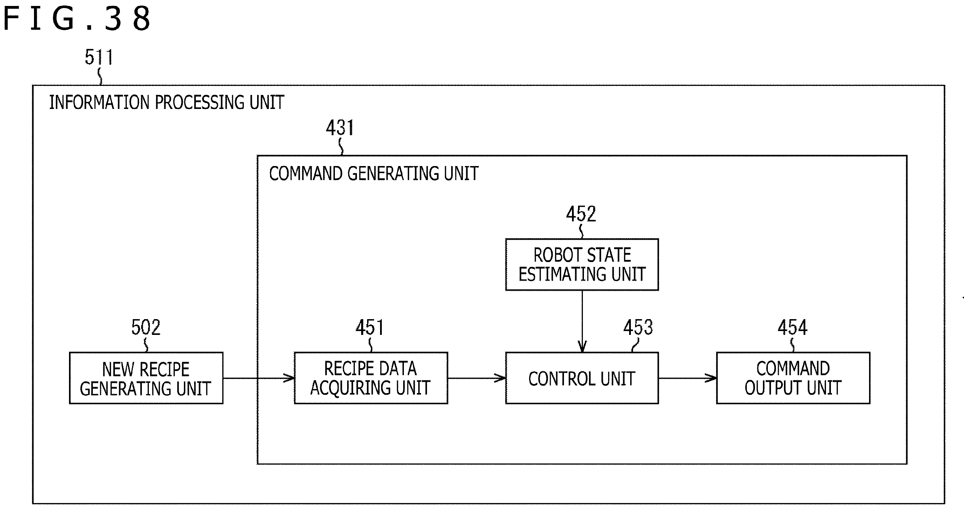

FIG. 38 is a diagram illustrating an exemplary system configuration.

[FIG. 39]

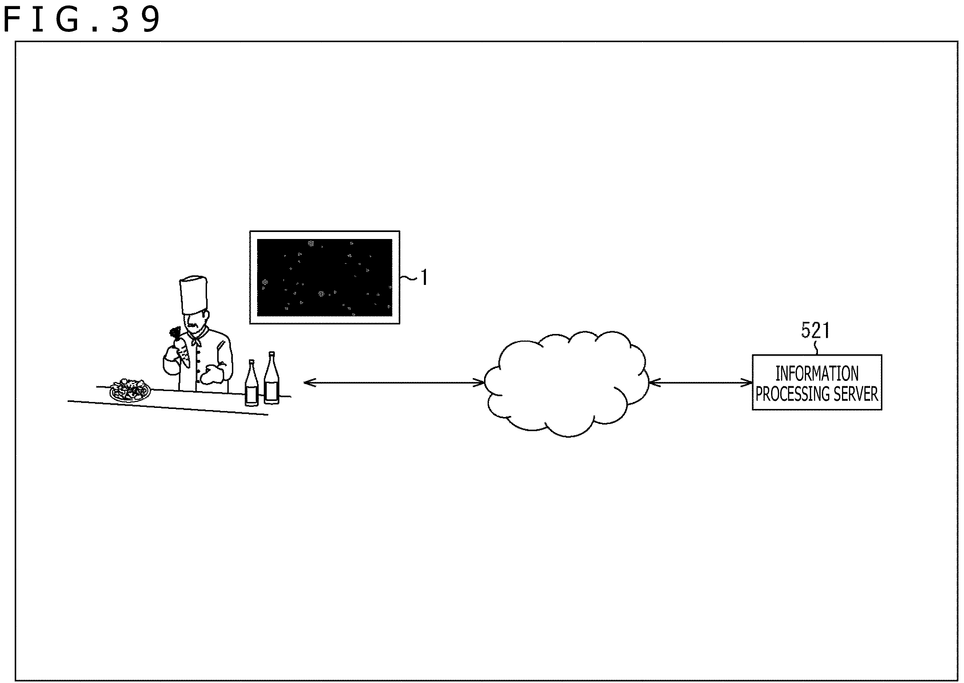

FIG. 39 is a diagram illustrating a configuration example of an information processing system.

[Description of Embodiment]

<<Outline of Present Technology>>

The present technology simulates the affinity of ingredients in combination and presents the simulation result in conjunction with the action of a cook in a cooking process.

The cook can devise a new cooking recipe by referring to the simulation result. That is, the present technology is used by the cook as a tool for supporting new cooking recipe devising.

Now, a mode for carrying out the present technology is described. The following are described in order.

1. Presentation of Simulation Result to Chef Working on Devising Recipe
2. NEW Recipe Data Generation Using Simulation Result
3. Modified Example

<<1. Presentation of Simulation Result to Chef Working on Devising Recipe>>

<Cooking Simulator>

FIG. 1 is a diagram illustrating a cooking simulation device according to an embodiment of the present technology.

In the example of FIG. 1, a cooking simulation device 1 including a large display is installed on a wall near a chef (cook) who is the user.

The cooking simulation device 1 is an information processing device that is used when, for example, a chef in a kitchen is working on devising a recipe for a new dish by trying various ingredients or various cooking methods or tasting. The cooking simulation device 1 has the function of simulating the affinity of ingredients in combination and presenting the simulation result.

The affinity of ingredients in combination includes the degree of affinity of multiple ingredients in combination and the flavor of multiple ingredients in combination. Affinity is determined from the flavor of ingredients in combination (the flavor of an ingredient obtained by combining multiple ingredients).

Presentation includes at least one of visual presentation using screen display and auditory presentation using sound.

Note that, a dish means a product obtained as a result of cooking. Cooking means the process of making a dish or an action (task) for making a dish.

The cooking simulation device 1 is a device including a flat-panel TV-shaped housing in the example of FIG. 1 but may be a device including a housing in another form such as a wearable device or a portable device including a tablet device or a smartphone.

FIG. 2 is a diagram illustrating an exemplary simulation result presentation.

In the situation in the upper part of FIG. 2, the chef who has selected, as an ingredient to be used in cooking, carrots from various ingredients is thinking of ingredients that go well with carrots. The chef is working on devising a cooking recipe that uses carrots and ingredients that go well with carrots.

For example, in a case where the chef says "tell me ingredients that go well with carrots" as illustrated in the speech bubble #1, the cooking simulation device 1 simulates the affinity of carrots and another ingredient in combination as indicated by the arrow A1. On the display of the cooking simulation device 1, a screen indicating the simulation result is displayed.

The chef can realize that salmons go well with carrots by looking at the simulation result presented by the cooking simulation device 1, as illustrated in the speech bubble #2, for example. According to such a realization, the chef develops a new recipe that uses carrots and salmons as indicated by the arrow A2.

In such a way, the cooking simulation device 1 is used as a tool for supporting recipe devising when the chef devises a recipe with his/her creativity. The chef can devise a new recipe by referring to a simulation result presented by the cooking simulation device 1.

Since the chef can use his/her voice to perform an operation for requesting a simulation, the chef can cause the cooking simulation device 1 to perform a simulation and check the simulation result to devise a recipe while doing something by hand during cooking. That is, a simulation result is presented in conjunction with the action of the chef such as saying something. An operation for requesting a simulation may be an operation by hand such as operating a touch panel.

The chef can cause the cooking simulation device 1 to perform simulations successively with different reference ingredients to check the combination of various ingredients. In the simulation of FIG. 2, carrots serve as a reference ingredient.

A new recipe is generally devised as follows: various ingredients are actually cooked in combination, and whether an expected flavor is achieved is determined by tasting. Since the affinity of ingredients in combination is presented, the chef can check, before actually cooking, the degree of affinity and the flavor and then cook. Cooking ingredients after checking the degree of affinity and the flavor can reduce the waste of ingredients and thus lead to a reduction in what are generally called food loss and waste.

In a case where a flavor obtained as a result of actually cooking ingredients that the chef has selected by looking at a simulation result is not good, the chef can cause the cooking simulation device 1 to perform a flavor simulation again by saying "tell me a different combination," for example. A new recipe is devised with the interaction between the chef and the cooking simulation device 1.

FIG. 3 is a diagram illustrating another exemplary simulation result presentation.

The cooking simulation device 1 performs a simulation according to not only a voice request but also to the result of recognition of the action of the chef during cooking. The action of the chef is recognized by analyzing an image taken by a camera pointed at the chef cooking. The cooking simulation device 1 has installed thereon the function of recognizing the action of the chef.

For example, in a case where, as illustrated in the upper part of FIG. 3, the action of the chef holding a carrot with his/her hand while thinking of ingredients that go well with carrots is recognized, the affinity of carrots and another ingredient in combination is simulated, and the result is presented, as indicated by the arrow.

Such a simulation that is triggered by the action of the chef is repeated while the chef is cooking. For example, in a case where it is recognized that the chef is to cook another ingredient after the carrots, an affinity simulation is performed by changing the reference ingredient from carrots to the other ingredient.

Since multiple ingredients are generally used in cooking, without any explicit voice request from the chef, different simulation results are provided in conjunction with the action of the chef.


<Example of GUI>

FIG. 4 is a diagram illustrating an exemplary simulation screen.

The simulation screen is configured as a screen displaying a virtual space in a certain view in which spheres representing ingredients are arranged. A single sphere corresponds to a single ingredient. Ingredients include, in addition to plant source foods such as vegetables and fruits and animal source foods such as meat and fish, processed ingredients, seasoning, and drinks such as water and liquor.

At substantially the center of the simulation screen, a reference ingredient icon P1 representing a reference ingredient serving as a reference for combination with other ingredients is placed. In the example of FIG. 1, the reference ingredient icon P1 is an icon representing "Pinot Noir." The reference ingredient icon P1 includes the image of Pinot Noir and the characters "Pinot Noir" superimposed thereon.

The simulation screen illustrated in FIG. 4 is a screen that is displayed in a case where Pinot Noir is selected as a reference ingredient, in response to a voice request from the chef or according to the result of recognition of the action of the chef. In the case where Pinot Noir has been selected as a reference ingredient, the reference ingredient icon P1 is displayed as being moved from a distant location to be positioned at substantially the center of the simulation screen.

When the reference ingredient icon P1 is positioned, as illustrated in FIG. 5, appropriate ingredient icons that are icons representing appropriate ingredients are arranged in a ring around the reference ingredient icon P1 being the center. An appropriate ingredient is an ingredient that achieves an affinity satisfying predetermined conditions with a reference ingredient in combination and is hence regarded as going well with the reference ingredient.

Each appropriate ingredient icon is displayed as being moved from a distant location to be gathered around the reference ingredient icon P1 and then moved slowly around the reference ingredient icon P1. In the example of FIG. 5, appropriate ingredient icons P11-1 to P11-10 representing 10 types of appropriate ingredients are displayed as the icons of ingredients that go well with Pinot Noir in combination.

As described later, the cooking simulation device 1 has the database (DB) of ingredient affinity information that is information indicating the affinity of respective ingredients in combination. Appropriate ingredients are selected according to the ingredient affinity information stored in the DB provided in the cooking simulation device 1.

After the appropriate ingredient icons P11-1 to P11-10 have been positioned around the reference ingredient icon P1, as illustrated in FIG. 6, each icon is moved depending on the affinity of the ingredients. At least any of the reference ingredient icon and the multiple appropriate ingredient icons are moved depending on the affinity of the respective ingredients.

For example, the icons are moved such that ingredients that go well together are brought closer to each other and ingredients that do not go well together are separated away from each other. When the two icons collide with each other, the two icons are displayed as being combined or merged to form a single icon.

In the example of FIG. 6, the appropriate ingredient icon P11-1 and the appropriate ingredient icon P11-10 are displayed as being partially merged, the appropriate ingredient icon P11-3 and the appropriate ingredient icon P11-4 are displayed as being partially merged, and the appropriate ingredient icon P11-8 and the appropriate ingredient icon P11-9 are displayed as being partially merged. For example, the appropriate ingredient icon P11-1 is an icon representing "iberian ham," and the appropriate ingredient icon P11-10 is an icon representing "Blackberry."

Icons being displayed as being merged indicates that the ingredients represented by the merged icons go well in combination. That is, ingredients going well in combination is presented in a visually identifiable manner by icon movements and icon shape changes.

In contrast, the icons representing ingredients that do not go well in combination are moved away from each other. Ingredients not going well in combination is also presented in a visually identifiable manner by icon movements.

FIG. 7 is a diagram illustrating an exemplary simulation screen that is displayed after the screen of FIG. 6.

In the example of FIG. 7, the merging of the respective icons further proceeds, and the reference ingredient icon P1 is also displayed as being partially merged with, for example, the appropriate ingredient icon P11-1 and the appropriate ingredient icon P11-10 that have already been merged. Such display is provided in a case where, for example, an ingredient obtained by combining iberian ham and Blackberry and Pinot Noir that is a reference ingredient go well together.

Since the affinity of ingredients is determined by the flavor as described above, depending on the flavor of a combined ingredient obtained by combining multiple ingredients, the ingredients that have been displayed so far may include some ingredients that go well with the combined ingredient and some ingredients that do not go well with the combined ingredient. That is, in a case where icons are merged, of the icons that have been displayed so far, some icons are brought closer to the merged icons and some icons are separated away from the merged icons.

In such a way, in a simulation screen, the affinity of respective ingredients in combination is displayed in a dynamically changing manner.

From the display of FIG. 7, the chef can confirm that iberian ham and Blackberry may be used in combination with Pinot Noir selected as a reference ingredient.

FIG. 8 is a diagram illustrating icons being merged, in an enlarged manner.

Partially merged icons illustrated in the upper part of FIG. 8 are an icon P21-1 representing Nori (seaweed) and an icon P21-2 representing chocolate. The icon P21-1 includes the image of seaweed, and the icon P21-2 includes the image of chocolate.

In a case where seaweed and chocolate go well in combination, on a simulation screen, the icon P21-1, and the icon P21-2 are displayed as being merged.

When the merging of the icon P21-1 and the icon P21-2 proceeds, as illustrated in the lower part of FIG. 8, the combined ingredient of seaweed and chocolate is displayed as a single icon P22. The icon P22 includes a composite image obtained by combining the image of seaweed and the image of chocolate.

In such a way, the merging of icons proceeds, and a combined ingredient obtained by combining multiple ingredients is eventually represented by a single icon.

The composite image of the icon P22 represents the flavor of seaweed and chocolate in combination. For example, the combined ingredient of seaweed and chocolate includes the flavor of seaweed and the flavor of chocolate. The composite image of the icon P22 is generated by blending the image of seaweed and the image of chocolate at a ratio based on the strengths of the respective flavors. For example, in a case where the flavor of chocolate is stronger than the flavor of seaweed in the flavor of the combined ingredient, the icon P22 includes a composite image with a strong chocolate image element.

Since the icon of a combined ingredient includes a composite image, the flavor of multiple ingredients in combination is presented in a visually identifiable manner. The chef can check the flavor of the combined ingredient by the displayed composite image of the icon.

In a case where multiple ingredients are combined, other than the flavor of each ingredient, a new flavor may be generated. In such a case, the icon is displayed with a change that expresses the newly generated flavor. For example, the newly generated flavor is expressed by the color of the icon, the shape of the icon, or the like.

In a case where the icons of certain ingredients are merged, when there are ingredients that do not go well with the combined ingredient, the icons of the ingredients that do not go well with the combined ingredient may be eliminated from the simulation screen.

Further, icons once merged may be displayed as being separated from each other. Icons are separated from each other in a case where, for example, multiple ingredients corresponding to merged icons include an ingredient that goes better with an ingredient corresponding to an icon at a distant location than with the merged ingredients.

With a simulation screen displayed in a dynamically changing manner in such a way, the chef can check ingredients that go well in combination and the flavor of the combination.

Since affinity is determined by flavor, the affinity of ingredients in combination is presented in conjunction with the flavors of ingredients or the flavor of ingredients in combination. A reference ingredient and appropriate ingredients may be ingredients described in a recipe prepared by the chef or new ingredients not described in the recipe. A recipe also includes recipe data that is used for controlling a cooking robot, which is described later.

Further, according to a simulation result, the affinity of a combination that the chef cooking has not recognized may be presented. In this case, the cooking simulation device 1 has data regarding the recipe prepared by the chef. The combination of ingredients not described in the recipe provided in the cooking simulation device 1 is selected as a combination that the chef has not recognized, for example.

With this, the chef can check the affinity of a combination that he/she has not realized.

<Presentation of Cooking Result>

In addition to the affinity of ingredients in combination, the cooking simulation device 1 simulates the state of a cooked ingredient and presents the simulation result.

FIG. 9 is a diagram illustrating an exemplary simulation result presentation.

In a case where, as illustrated in the upper part of FIG. 9, the cooking simulation device 1 recognizes that the chef has put potatoes in the pot, the state of the ingredient, namely, the potatoes, simmered in the pot is simulated, and the result is presented, as indicated by the arrow.

The chef can check, by looking at the simulation result presented by the cooking simulation device 1, an appropriate length of time for simmering the potatoes, an appropriate strength of fire for simmering the potatoes, and the like. In this case, the cooking simulation device 1 has information indicating how each ingredient changes when being cooked by various methods.

Since a single dish is generally completed through multiple cooking processes, a simulation result is provided in a dynamically changing manner in conjunction with the action of the chef.

In such a way, the cooking simulation device 1 can perform various simulations related to cooking other than the affinity of ingredients in combination. For example, in the situation of FIG. 9, not the state of the ingredient, but the flavor of the ingredient obtained by cooking may be simulated, and the simulation result may be presented.

<Ingredient Affinity Information>

FIG. 10 is a diagram illustrating an example of an ingredient affinity information DB.

An ingredient affinity information DB 11 is the DB of ingredient affinity information. The ingredient affinity information DB 11 is provided in the cooking simulation device 1, for example.

The ingredient affinity information DB 11 stores ingredient affinity information that is information indicating the affinity of each ingredient and another ingredient. As illustrated in the speech bubble of FIG. 10, for example, ingredient affinity information regarding an ingredient A includes information indicating the affinity of the ingredient A and an ingredient B in combination, information indicating the affinity of the ingredient A and an ingredient C in combination, and the like. Ingredient affinity information regarding the ingredient B and the ingredient C also includes information indicating the affinity with another ingredient in combination.

The affinity of ingredients is represented by a numerical value, for example. In this case, the higher the numerical value of ingredient affinity information, the better ingredients go in combination, and the lower the numerical value of ingredient affinity information, the worse ingredients go in combination.

In a case where a certain ingredient is selected as a reference ingredient, for example, ingredients having ingredient affinity information with numerical values equal to or larger than a threshold are selected as appropriate ingredients and presented as described above. The conditions on ingredient affinity information for appropriate ingredients are set in advance, and ingredients satisfying the conditions are selected as appropriate ingredients.

A numerical value indicating the affinity of ingredients is set by a person in his/her experience or set by a person actually eating a combined ingredient and evaluating the affinity, for example.

As described later, in a case where information indicating the characteristics of ingredients such as flavor and chemical structures is provided, a numerical value indicating the affinity of ingredients may be obtained and set according to the information indicating the characteristics of ingredients. In this case, the information indicating the characteristics of each ingredient is analyzed or a calculation based on the information indicating the characteristics of each ingredient is performed to obtain a numerical value indicating the affinity of ingredients.

In a case where various cooking recipes are provided, the affinity of ingredients may be obtained according to the recipes. In each recipe, ingredients to be used are described. For example, ingredients that are often used in combination are set as ingredients that go well in combination.

In such a way, the affinity of ingredients may be determined according to other criteria.

Further, information indicating the affinity of a combined ingredient obtained by combining a certain ingredient and another ingredient and each ingredient may be stored in the ingredient affinity information DB 11 as ingredient affinity information.

Information indicating the affinity of a cooked ingredient obtained by cooking a certain ingredient by various methods and each ingredient may be stored in the ingredient affinity information DB 11 as ingredient affinity information.

Whether ingredients are determined as going well together or not varies depending on nationality, region, culture, or the like. The ingredient affinity information DB 11 may be provided for each of different attributes of ingredients such as nationality, region, and culture.

The ingredient affinity information DB 11 may be provided for each category of ingredients such as chocolate, pure rice sake, and fruit liqueur.

FIG. 11 is a diagram illustrating exemplary components of flavor.

Deliciousness that people feel in the brain, that is, "flavor," mainly includes, as illustrated in FIG. 11, the combination of taste obtained by the sense of taste of people, aroma obtained by the sense of smell of people, and texture obtained by the sense of touch of people.

The affinity of ingredients in combination is determined by the taste of ingredients in combination, the aroma of ingredients in combination, and the texture of ingredients in combination. The affinity of ingredients in combination may be determined by at least one of taste, aroma, and texture.

FIG. 12 is a diagram illustrating exemplary information that is used for a simulation.

In the simulation of the affinity of ingredients in combination, other than the ingredient affinity information, flavor subjective information stored in a flavor subjective information DB 21, sensing information stored in a sensing information DB 22, and chemical structure information stored in a chemical structure information DB 23 are used.

Since the affinity of ingredients in combination includes the degree of affinity of ingredients and the flavor of ingredients in combination, the ingredient affinity information is mainly used for identifying the degree of affinity of ingredients. Further, the flavor subjective information, the sensing information, and the chemical structure information are mainly used for identifying the flavor of ingredients in combination.

In a case where the flavor of ingredients in combination is identified in reference to the flavor subjective information and the sensing information, the cooking simulation device 1 presents the affinity of the ingredients in combination by using the flavor subjective information, the sensing information, and the ingredient affinity information.

Further, in a case where the flavor of ingredients in combination is identified in reference to the flavor subjective information, the sensing information, and the chemical structure information, the cooking simulation device 1 presents the affinity of the ingredients in combination by using the flavor subjective information, the sensing information, the chemical structure information, and the ingredient affinity information.

The four types of DBs, namely, the ingredient affinity information DB 11, the flavor subjective information DB 21, the sensing information DB 22, and the chemical structure information DB 23, may be provided in the cooking simulation device 1 or a server on the Internet. The four types of DBs may be provided in the cooking simulation device 1 and the server on the Internet in a distributed manner.

FIG. 13 is a diagram illustrating exemplary information that is stored in the flavor subjective information DB 21.

The flavor subjective information DB 21 is the database of subjective evaluations related to the flavors of respective ingredients. For example, a large number of people are asked to eat each ingredient, and information regarding the flavor subjective evaluation values expressed in a predetermined scale is stored in the flavor subjective information DB 21, as flavor subjective information.

For example, flavor subjective information regarding the ingredient A and the ingredient B is represented by an (e-dimensional) vector representation in a flavor subjective evaluation space E as in Expression (1) below.
[Math. 1]

$$E \ (\text{ingredient A}) = [EA1, \ EA2, \ ... \ EAi]$$

$$E \ (\text{ingredient B}) = [EB1, \ EB2, \ ... \ EBi] \ \cdots \ (1)$$

Flavor subjective information regarding another ingredient is also represented by a vector having, as its element, each item of the subjective evaluation. For example, EAi is a coefficient corresponding to the evaluation value of the item of an index i included in the subjective evaluation of the ingredient A.

FIG. 14 is a diagram illustrating exemplary information that is stored in the sensing information DB 22.

The sensing information DB 22 is the database of sensing results of the flavors of respective ingredients. For example, in a cooking process, the flavor of each ingredient is measured by a flavor measuring instrument, and the sensing information (sensor information) obtained as a result of the measurement is stored in the sensing information DB 22.

Since flavor is represented by taste, aroma, and texture, the flavor of each ingredient is measured using a taste measuring instrument, an aroma measuring instrument, a texture measuring instrument, and the like. Note that, texture includes the elasticity, viscosity, temperature, or the like of an ingredient.

Sensing information regarding the ingredient A and the ingredient B is represented by an (s-dimensional) vector representation in a sensing information space S as in Expression (2) below.

[Math. 2]

$$S \ (\text{ingredient A}) = [SA1, \ SA2, \ ... \ SAi]$$

$$S \ (\text{ingredient B}) = [SB1, \ SB2, \ ... \ SBi] \ \cdots \ (2)$$

Sensing information regarding another ingredient is also represented by a vector having each item as its element. For example, SAi is a coefficient corresponding to the sensor value of the item of the index i included in the sensing information regarding the ingredient A.

FIG. 15 is a diagram illustrating exemplary information that is stored in the chemical structure information DB 23.

The chemical structure information DB 23 is the database of the chemical structures of respective ingredients. For example, the chemical structure of each ingredient is measured by a separation analyzer utilizing the principle of chromatography, and the chemical structure information obtained as a result of the measurement is stored in the chemical structure information DB 23.

Chemical structure information regarding the ingredient A and the ingredient B is represented by a (c-dimensional) vector representation in a chemical structure space Ch with use of molecular descriptors as in Expression (3) below.

[Math. 3]

$$Ch \ (\text{ingredient A}) = [NA1, \ NA2, \ ... \ NAi]$$

$$Ch \ (\text{ingredient B}) = [NB1, \ NB2, \ ... \ NBi] \ \cdots \ (3)$$

Chemical structure information regarding another ingredient is also represented by a vector having, as its element, each chemical substance that is used for expressing chemical structures. For example, NAi is a coefficient corresponding to the amount of a chemical substance of the index i included in the ingredient A. The ingredients A and B each have a chemical substance that only the ingredient A or the ingredient B has, and the coefficient of such a chemical substance is 0.

The chemical structure of each ingredient may be expressed using another description technique such as structural descriptors or count descriptors. Not only chemical structures but also information indicating other chemical features such as properties or reaction may be provided in the chemical structure information DB 23.

<Configuration Example of Cooking Simulation Device>

FIG. 16 is a block diagram illustrating a configuration example of the hardware of the cooking simulation device 1.

As illustrated in FIG. 16, the cooking simulation device 1 includes a computer. A CPU (Central Processing Unit) 101, a ROM (Read Only Memory) 102, and a RAM (Random Access Memory) 103 are connected to each other by a bus 104.

The CPU 101 loads a program stored in a storage unit 111, for example, on the RAM 103 through an input-output interface 105 and the bus 104 to execute the program and thereby performs various types of processing such as cooking-related simulations.

To the bus 104, the input-output interface 105 is connected. To the input-output interface 105, a microphone 106, a camera 107, an operation unit 108, a display 109, a speaker 110, the storage unit 111, a communication unit 112, and a drive 113 are connected.

The microphone 106 detects the voice of the chef and outputs the voice data to the CPU 101.

The camera 107 captures the cooking simulation device 1 and its surroundings including the action of the chef. Data regarding the image captured by the camera 107 is supplied to the CPU 101.

The operation unit 108 includes a touch panel provided on the display 109, buttons provided on the housing of the cooking simulation device 1, or the like. The operation unit 108 detects the operation of the chef and outputs information indicating the content of the operation to the CPU 101. The cooking simulation device 1 may be operated by the touch panel or the buttons instead of voice.

The display 109 includes an LCD, an organic EL display, or the like. The display 109 displays a simulation screen under the control of the CPU 101.

The speaker 110 outputs synthesized voice under the control of the CPU 101. A simulation result may be presented by voice output from the speaker 110.

The storage unit 111 includes a hard disk, a nonvolatile memory, or the like. The storage unit 111 stores various types of information such as programs that the CPU 101 executes. The ingredient affinity information DB 11, the flavor subjective information DB 21, the sensing information DB 22, and the chemical structure information DB 23 may be constructed in the storage unit 111.

The communication unit 112 includes a network interface or the like. The communication unit 112 communicates with the server on the Internet. The communication unit 112 receives information transmitted from the server and outputs the information to the CPU 101, to transmit, to the server, information supplied from the CPU 101.

The drive 113 drives a removable medium 114 to perform data reading from the removable medium 114 and data writing to the removable medium 114.

FIG. 17 is a block diagram illustrating a functional configuration example of the cooking simulation device 1.

As illustrated in FIG. 17, the cooking simulation device 1 realizes a simulation subject recognizing unit 131, a simulation calculation unit 132, and a presentation unit 133. At least some of the functional units illustrated in FIG. 17 are realized by the CPU 101 of FIG. 16 executing a predetermined program.

The simulation subject recognizing unit 131 analyzes voice data supplied from the microphone 106 or image data supplied from the camera 107, to recognize a simulation subject.

For example, in a case where the simulation subject recognizing unit 131 identifies, as a result of analyzing voice data, that the chef has said "tell me ingredients that go well with carrots," the simulation subject recognizing unit 131 recognizes that the simulation of the affinity of ingredients in combination is to be performed using carrots as a reference ingredient.

Also in a case where the simulation subject recognizing unit 131 identifies, as a result of analyzing image data, that the chef is thinking of ingredients that go well with carrots, the simulation subject recognizing unit 131 recognizes that the simulation of the affinity of ingredients in combination is to be performed using carrots as a reference ingredient.

In a case where the simulation subject recognizing unit 131 identifies, as a result of analyzing image data, that the chef has put potatoes in the pot, the simulation subject recognizing unit 131 recognizes that the simulation of a change in state of the ingredient is to be performed.

The simulation subject recognizing unit 131 outputs information indicating the recognized simulation subject, to the simulation calculation unit 132.

The simulation calculation unit 132 simulates a subject recognized by the simulation subject recognizing unit 131.

In a case where the simulation calculation unit 132 simulates the affinity of carrots serving as a reference ingredient and another ingredient in combination, the simulation calculation unit 132 selects appropriate ingredients in reference to the ingredient affinity information.

For example, an ingredient having, as its ingredient affinity information indicating the affinity with carrots, a numerical value equal to or larger than the threshold is selected as an appropriate ingredient. The simulation calculation unit 132 identifies the movement of the reference ingredient icon and each appropriate ingredient icon, in reference to the ingredient affinity information.

Further, the simulation calculation unit 132 simulates the flavor of carrots serving as a reference ingredient and each appropriate ingredient in combination and the flavor of the appropriate ingredients in combination.

The flavor of ingredients in combination is simulated in reference to the sensing information and the flavor subjective information. For example, the simulation calculation unit 132 performs a predetermined calculation such as a vector calculation in reference to sensing information and flavor subjective information regarding ingredients to be combined, and thereby identifies the flavor of the ingredients in combination.

Flavor can be simulated using, instead of both the sensing information and the flavor subjective information, at least any of the sensing information, the flavor subjective information, and the chemical structure information.

Further, in a case where the simulation calculation unit 132 simulates a change in state of an ingredient, the simulation calculation unit 132 simulates a change in flavor in reference to information stored in an unillustrated DB. The cooking simulation device 1 has, as a DB that the simulation calculation unit 132 is accessible, the DB of information indicating a change in flavor that occurs in a case where each ingredient is cooked by various methods.

The simulation calculation unit 132 outputs information indicating the simulation result, to the presentation unit 133.

The presentation unit 133 controls the display 109 to display a simulation screen according to the result of a simulation by the simulation calculation unit 132.

Further, in a case where the presentation unit 133 presents a simulation result by synthesized voice, the presentation unit 133 outputs, according to the result of a simulation by the simulation calculation unit 132, voice indicating the simulation result from the speaker 110.

<Operation of Cooking Simulation Device>

With reference to the flowchart of FIG. 18, cooking simulation processing by the cooking simulation device 1 is described.

In Step S1, the microphone 106 detects the voice of the chef.

In Step S2, the camera 107 captures the cooking simulation device 1 and its surroundings including the action of the chef.

In Step S3, the simulation subject recognizing unit 131 analyzes voice data supplied from the microphone 106 or image data supplied from the camera 107, to recognize a simulation subject.

In Step S4, the simulation calculation unit 132 simulates the subject recognized by the simulation subject recognizing unit 131.

In Step S5, the presentation unit 133 controls the display 109 to present the simulation result to the chef.

After that, the processing returns to Step S1, and the processing described above is repeated. The processing of FIG. 18 is repeated during cooking, for example.

With the processing described above, the cooking simulation device 1 can dynamically present, to the chef working on devising a new recipe, the affinity of respective ingredients in combination and the flavor of the respective ingredients in combination. That is, the cooking simulation device 1 can appropriately present the affinity of ingredients in combination.

The chef can develop a new recipe by referring to the affinity of ingredients in combination.

<Example of Dynamically Generating Ingredient Affinity Information>

Although the cooking simulation device 1 has the ingredient affinity information DB 11 having stored therein the ingredient affinity information, ingredient affinity information may be generated while the chef is working on devising a new recipe during cooking.

In this case, in reference to ingredient affinity information generated during cooking, the affinity of ingredients in combination is simulated, and the simulation result is presented during cooking. Ingredient affinity information is dynamically generated depending on the action of the chef, and a simulation is performed in reference to the generated ingredient affinity information.

FIG. 19 is a diagram illustrating an exemplary simulation result presentation.

In the situation in the upper part of FIG. 19, the chef is working on a recipe while cooking. Around the chef, the flavor measuring instrument for detecting the flavor of an ingredient used in cooking is provided. The flavor measuring instrument measures, during cooking, data configuring the flavor of an ingredient that the chef is cooking.

Data indicating the result of the measurement by the flavor measuring instrument is transmitted to the cooking simulation device 1. As the data indicating the measurement result, taste data indicating a taste measurement result, aroma data indicating an aroma measurement result, and texture data indicating a texture measurement result are transmitted.

The cooking simulation device 1 generates sensing information according to the data transmitted from the flavor measuring instrument. As described above, sensing information is information indicating the flavors of ingredients.

Further, the cooking simulation device 1 generates ingredient affinity information in reference to the sensing information. The ingredient affinity information generated here is information indicating the affinity of the flavor of the ingredient used in cooking and the flavor of another ingredient.

The affinity of ingredients is obtained by performing a predetermined calculation such as a vector calculation in reference to the sensing information. For example, the result of a calculation based on the sensing information regarding the ingredient used in cooking and sensing information regarding an ingredient stored in the sensing information DB 22 is generated as ingredient affinity information indicating the affinity of the ingredient used in cooking and another ingredient in combination.

After the ingredient affinity information has been generated, the affinity of ingredients in combination is simulated using the ingredient used in cooking as a reference ingredient, and the simulation result is presented, as indicated by the arrow.

The chef who has checked the simulation result presented by the cooking simulation device 1 can check ingredients that go well with the ingredient used in cooking and devise a new recipe.

FIG. 20 is a diagram illustrating a kitchen and its surroundings in an enlarged manner.

Around the kitchen where the chef is cooking, various apparatuses of the flavor measuring instrument are provided. Some of the apparatuses are attached to the body of the chef.

Each apparatus provided around the kitchen is connected to the cooking simulation device 1 via wired or wireless communication. Each apparatus provided around the kitchen may be connected to the cooking simulation device 1 via a network.

As illustrated in FIG. 20, an aroma sensor 141 is attached to the upper body of the chef. The aroma sensor 141 measures the aroma of an ingredient used in cooking and transmits the aroma data to the cooking simulation device 1.

A taste sensor 142 is provided on the top board in the kitchen. The taste sensor 142 measures the taste of an ingredient and transmits the taste data to the cooking simulation device 1.

The taste sensor 142 is used with a sensor unit 142A, which is provided at the distal end of the cable, in contact with an ingredient used in cooking, as illustrated in FIG. 21. In a case where the taste sensor 142 is an artificial lipid membrane taste sensor, the sensor unit 142A is provided with a lipid membrane.

Not only taste data but also texture data may be measured by the taste sensor 142 and transmitted to the cooking simulation device 1. In this case, the taste sensor 142 has the function of a texture sensor. For example, the taste sensor 142 measures texture data regarding polymer content, moisture content, oil content, or the like.

In such a way, the aroma sensor 141 and the taste sensor 142 are provided as the flavor measuring instrument, for example. Around the kitchen, various apparatuses other than the apparatus illustrated in FIG. 20 are appropriately provided.

FIG. 22 is a block diagram illustrating a configuration example of the cooking simulation device 1.

Of the components illustrated in FIG. 22, the components that are the same as the components described above are denoted by the same reference signs. The redundant description is appropriately omitted. The components illustrated in FIG. 22 are provided in addition to the components described with reference to FIG. 16 and FIG. 17.

The cooking simulation device 1 realizes a sensing information generating unit 161, a flavor subjective information generating unit 162, and an ingredient affinity information generating unit 163 by the CPU 101 (FIG. 16) executing a predetermined program. The sensing information generating unit 161 receives data output from the aroma sensor 141, the taste sensor 142, and the texture sensor 143.

The texture sensor 143 includes a sensor configured to output various types of data that are used for analyzing texture, such as a hardness sensor, a stress sensor, a water content sensor, or a temperature sensor. A hardness sensor, a stress sensor, a water content sensor, or a temperature sensor may be provided to a cooking tool such as a kitchen knife, a frying pan, or an oven.

The sensing information generating unit 161 generates sensing information indicating the flavor of an ingredient used in cooking, in reference to aroma data transmitted from the aroma sensor 141, taste data transmitted from the taste sensor 142, and texture data transmitted from the taste sensor 142 or the texture sensor 143. The sensing information generated by the sensing information generating unit 161 is supplied to the flavor subjective information generating unit 162 and the ingredient affinity information generating unit 163.

The flavor subjective information generating unit 162 generates flavor subjective information in reference to sensing information supplied from the sensing information generating unit 161. In the flavor subjective information generating unit 162, information that is used for converting sensing information into flavor subjective information is provided in advance. The flavor subjective information generating unit 162 generates flavor subjective information by converting sensing information into flavor subjective information.

An inference model such as a neural network whose input is sensing information and output is flavor subjective information may be learned in advance to be provided in the flavor subjective information generating unit 162. In this case, the flavor subjective information generating unit 162 inputs sensing information supplied from the sensing information generating unit 161 to the inference model and generates flavor subjective information as the output of the inference model. The flavor subjective information generated by the flavor subjective information generating unit 162 is supplied to the ingredient affinity information generating unit 163.

Flavor subjective information may be generated in reference to a flavor subjective evaluation input by the chef at the timing of tasting. In this case, the chef tastes an ingredient used in cooking and inputs the flavor that he/she has felt.

A biomedical sensor may be attached to the body of the chef cooking, and flavor subjective information may be generated according to a biomedical reaction measured by the biomedical sensor when the chef tastes an ingredient.

The ingredient affinity information generating unit 163 generates, in reference to sensing information regarding an ingredient used in cooking supplied from the sensing information generating unit 161 and the sensing information regarding each ingredient stored in the sensing information DB 22, ingredient affinity information indicating the affinity of the ingredient used in cooking and another ingredient. The ingredient affinity information is generated by performing a predetermined calculation such as a vector calculation in reference to the sensing information.

Further, the ingredient affinity information generating unit 163 generates, in reference to flavor subjective information regarding an ingredient used in cooking supplied from the flavor subjective information generating unit 162 and the flavor subjective information regarding each ingredient stored in the flavor subjective information DB 21, ingredient affinity information indicating the affinity of the ingredient used in cooking and another ingredient. The ingredient affinity information is generated by performing a predetermined calculation such as a vector calculation in reference to the flavor subjective information.

The ingredient affinity information generating unit 163 may generate ingredient affinity information by using flavor subjective information and sensing information in combination. The ingredient affinity information is generated using at least one of the flavor subjective information and the sensing information.

Ingredient affinity information may be generated using flavor subjective information, sensing information, and chemical structure information in combination. The ingredient affinity information is generated using at least any of the flavor subjective information, the sensing information, and the chemical structure information.

The ingredient affinity information generated by the ingredient affinity information generating unit 163 is supplied to the simulation calculation unit 132 (FIG. 17) as ingredient affinity information regarding a reference ingredient and used for the simulation of the affinity of ingredients in combination.

In a case where ingredient affinity information is generated in reference to flavor subjective information and sensing information regarding an ingredient used in cooking, the cooking simulation device 1 presents the affinity of ingredients in combination by using the flavor subjective information, the sensing information, and the ingredient affinity information.

Depending on the action of the chef in a cooking process, there occur changes in flavor subjective information and sensing information. Changes in flavor subjective information and sensing information lead to a dynamic change in simulation result.

The chef can think of the combination of ingredients or the like by looking at a simulation result provided in a dynamically changing manner and develop a new recipe.

<<2. NEW Recipe Data Generation Using Simulation Result>>

In response to an instruction from the chef to whom a simulation result has been presented by the cooking simulation device 1, recipe data that is data regarding a recipe that is used for controlling the cooking robot may be generated.

<NEW Recipe Data Generation>

FIG. 23 is a diagram illustrating an exemplary flow of NEW recipe data generation.

As indicated by the arrow A1, recipe data regarding a certain dish is input to the cooking simulation device 1. The cooking simulation device 1 regards the input recipe data as original recipe data and updates the content of the original recipe data to generate new recipe data (NEW recipe data).

Recipe data is data prepared for each dish. In recipe data, ingredients that are used in respective cooking processes for completing a dish are described.

The cooking simulation device 1 selects, as a reference ingredient, any of ingredients described in the recipe data as ingredients to be used in the cooking processes. The ingredients described in the recipe data may be presented to the chef, and an ingredient to be used as a reference ingredient may be selected by the chef.

When a reference ingredient is selected, the affinity of the reference ingredient and another ingredient in combination is simulated, and the simulation result is presented, as indicated by the arrow A2. As the appropriate ingredients, for example, ingredients not described in the recipe data, that is, ingredients not supposed to be used in the cooking processes in the original recipe data, are selected.

The chef can check, as ingredients that go well with the reference ingredient, the ingredients not described in the recipe data, by looking at the simulation result presented by the cooking simulation device 1.

In a case where the chef says "use salmons" or the like to select a predetermined ingredient from the appropriate ingredients, the content of the recipe data is updated such that the ingredient selected by the chef is used in the cooking processes, as indicated by the arrow A3. The recipe data having the updated content is transmitted as NEW recipe data to the server configured to manage recipe data, for example, as indicated by the arrow A4.

The chef can think of the combination of ingredients or the like while looking at the simulation result and generate new recipe data in which the newly devised content is reflected.

<Configuration of Control System>

FIG. 24 is a diagram illustrating a configuration example of a control system using recipe data.

The control system includes a data processing device 301 and a cooking robot 302. The cooking robot 302 is a robot including drive system devices such as cooking arms and various sensors and having a cooking function. The cooking robot 302 is installed in a store such as a restaurant or home.

The data processing device 301 is a device configured to control the cooking robot 302. The data processing device 301 includes a computer or the like.

As illustrated in the left end of FIG. 24, the cooking robot 302 is controlled by the data processing device 301 according to recipe data. In a case where the data processing device 301 acquires NEW recipe data newly generated by the cooking simulation device 1, the cooking robot 302 is controlled according to the NEW recipe data. In the following, recipe data includes original recipe data managed in the server or the like and NEW recipe data generated by the cooking simulation device 1.

For example, in a case where recipe data is input as indicated by the arrow A1, the data processing device 301 outputs an instruction command according to the description of the recipe data, as indicated by the arrow A2, to control the cooking operation of the cooking robot 302.

The cooking robot 302 drives each unit such as the cooking arm according to the instruction command supplied from the data processing device 301, to perform the cooking operation of each cooking process. An instruction command includes, for example, information for controlling the torque, drive direction, and drive amount of the motor provided to the cooking arm.

Until the dish is completed, instruction commands are sequentially output from the data processing device 301 to the cooking robot 302. The cooking robot 302 performs operations based on the instruction commands to eventually complete the dish.

FIG. 25 is a diagram illustrating exemplary content described in recipe data.

As illustrated in FIG. 25, a single piece of recipe data includes multiple cooking process data sets. In the example of FIG. 25, a cooking process data set related to a cooking process #1, a cooking process data set related to a cooking process #2, and a cooking process data set related to a cooking process #N are included.

Each cooking process data set includes cooking operation information that is information regarding a cooking operation for realizing the cooking process. For example, a single cooking process data set includes time series data regarding cooking operation information for realizing a single cooking process.

Cooking operation information includes ingredient information and operation information.

Ingredient information is information regarding ingredients that are used in a cooking process. Information regarding ingredients includes information indicating the types of ingredients, the amount of ingredients, the sizes of ingredients, and the like.

Note that, ingredients include, not only ingredients that have not been cooked at all, but also cooked (prepared) ingredients that have been subjected to a certain cooking process. Ingredient information included in cooking operation information regarding a certain cooking process includes information regarding ingredients subjected to the preceding cooking processes.

Operation information is information regarding the motion of the cooking arms or the like in a cooking process. Information regarding motion includes information indicating the types of cooking tools to be used in cooking or the like.

For example, operation information regarding a cooking process in which a certain ingredient is cut includes information indicating that a kitchen knife is used as a cooking tool and information indicating cutting positions, the number of cuts, cutting force, angle, and speed, and the like.

Further, operation information regarding a cooking process in which liquid put in a pot as an ingredient is stirred includes information indicating that a ladle is used as a cooking tool and information indicating stirring force, angle, speed, and time and the like.

Operation information regarding a cooking process in which a certain ingredient is baked in an oven includes information indicating that an oven is used as a cooking tool and information indicating the temperature of the oven, baking time, and the like.

Operation information regarding a food layout cooking process includes food layout method information indicating tableware to be used for food layout, the arrangement of ingredients, the colors of ingredients, and the like.

FIG. 26 is a diagram illustrating an exemplary flow of dish reproduction based on recipe data.

As illustrated in FIG. 26, the cooking robot 302 reproduces a dish by repeating, by the cooking process, cooking based on the cooking operation information at each time point included in the cooking process data sets described in the recipe data. A single dish is completed through multiple cooking processes, namely, the cooking processes #1 to #N.

FIG. 27 depicts diagrams illustrating a placement example of the data processing device 301.

As illustrated in A of FIG. 27, the data processing device 301 is provided outside the cooking robot 302, for example. In the example of A of FIG. 27, the data processing device 301 and the cooking robot 302 are connected to each other via a network such as the Internet.

An instruction command transmitted from the data processing device 301 is received by the cooking robot 302 via the network. From the cooking robot 302 to the data processing device 301, various types of data such as an image taken by the camera of the cooking robot 302 and sensor data measured by the sensor provided to the cooking robot 302 are transmitted via the network.

As illustrated in B of FIG. 27, the data processing device 301 may be provided inside the housing of the cooking robot 302. In this case, the operation of each unit of the cooking robot 302 is controlled according to an instruction command generated by the data processing device 301.

Now, a case where the data processing device 301 is provided outside the cooking robot 302 is mainly described.

<Outer Appearance of Cooking Robot>

FIG. 28 is a perspective view illustrating the outer appearance of the cooking robot 302.

As illustrated in FIG. 28, the cooking robot 302 is a kitchen robot including a housing 311 in a horizontally long rectangular parallelepiped shape. Various components are provided inside the housing 311 serving as the main body of the cooking robot 302.

On the rear side of the housing 311, a cooking assistance system 312 is provided. The spaces formed in the cooking assistance system 312 being partitioned by the thin plate members have the function of assisting cooking with cooking arms 321-1 to 321-4, such as the function of a refrigerator, a microwave oven, or a storage.

A rail is provided in a top board 311A in the longitudinal direction, and the cooking arms 321-1 to 321-4 are provided in the rail. The cooking arms 321-1 to 321-4 can be repositioned along the rail serving as a movement mechanism.

The cooking arms 321-1 to 321-4 are robotic arms including cylindrical members connected to each other through joint portions. Various cooking-related tasks are performed with the cooking arms 321-1 to 321-4.

The space above the top board 311A is a cooking space where the cooking arms 321-1 to 321-4 cook.

Four cooking arms are illustrated in FIG. 28, but the number of cooking arms is not limited to four. In the following, unless there is a need to distinguish between the cooking arms 321-1 to 321-4, the cooking arms 321-1 to 321-4 are collectively referred to as a "cooking arm 321" as appropriate.

FIG. 29 is a diagram illustrating the cooking arms 321 in an enlarged manner.

As illustrated in FIG. 29, attachments having various cooking functions are attached to the distal ends of the cooking arms 321. As the attachments for the cooking arms 321, various attachments such as an attachment having a manipulator function (hand function) of gripping ingredients or tableware and an attachment having a knife function of cutting ingredients are provided.

In the example of FIG. 29, a knife attachment 331-1 that is an attachment having a knife function is attached to the cooking arm 321-1. A lump of meat placed on the top board 311A has been cut using the knife attachment 331-1.

A spindle attachment 331-2 that is an attachment used for fixing or rotating ingredients is attached to the cooking arm 321-2.

A peeler attachment 331-3 that is an attachment having a peeler function of peeling ingredients is attached to the cooking arm 321-3.

A potato lifted by the cooking arm 321-2 using the spindle attachment 331-2 is being peeled by the cooking arm 321-3 using the peeler attachment 331-3. In such a way, the multiple cooking arms 321 can perform a single task in cooperation with each other.

A manipulator attachment 331-4 that is an attachment having the manipulator function is attached to cooking arm 321-4. With the use of the manipulator attachment 331-4, a frying pan in which chicken has been put is being transported to the space of the cooking assistance system 312 that has the oven function.

Cooking with such cooking arms 321 proceeds while the attachments are replaced as appropriate depending on the content of tasks. The manipulator attachment 331-4 can be attached to each of the four cooking arms 321, that is, the same attachment can also be attached to each of the multiple cooking arms 321.

The cooking robot 302 cooks not only with the above-mentioned attachments provided as tools for cooking arms, but also appropriate tools that are the same as tools that humans use in cooking. For example, with the manipulator attachment 331-4 griping a knife that humans use, ingredients are processed, for example, cut, using the knife.

<Configuration of Cooking Arm>

FIG. 30 is a diagram illustrating the outer appearance of the cooking arm 321.

As illustrated in FIG. 30, the cooking arm 321 generally includes the thin cylindrical members connected to each other through the hinge portions serving as the joint portions. Each hinge portion is provided with, for example, a motor configured to generate force for driving each member.

As the cylindrical members, an attaching/detaching member 351, a relay member 353, and a base member 355 are provided in order from the distal end.

The attaching/detaching member 351 and the relay member 353 are connected to each other through a hinge portion 352, and the relay member 353 and the base member 355 are connected to each other through a hinge portion 354.

An attaching/detaching portion 351A to/from which an attachment is attached and detached is provided at the distal end of the attaching/detaching member 351. The attaching/detaching member 351 has the attaching/detaching portion 351A to/from which various attachments are attached and detached and functions as a cooking function arm portion configured to cook by operating the attachment.

The rear end of the base member 355 is provided with an attaching/detaching portion 356 that is attached to the rail. The base member 355 functions as a movement function arm portion configured to achieve the movement of the cooking arm 321.

FIG. 31 is a diagram illustrating an exemplary range of motion of each portion of the cooking arm 321.

As indicated by the ellipse #1, the attaching/detaching member 351 is rotatable about the central axis of the circular cross section. The small flat circle illustrated in the center of the ellipse #1 indicates the direction of the rotational axis illustrated as the long dashed short dashed line.

As indicated by the circle #2, the attaching/detaching member 351 is rotatable about an axis passing through a fitting portion 351B for the hinge portion 352. Further, the relay member 353 is rotatable about an axis passing through a fitting portion 353A for the hinge portion 352.

The two small circles illustrated inside the circle #2 indicate the direction of the respective rotational axes (direction perpendicular to the drawing sheet). The range of motion of the attaching/detaching member 351 about the axis passing through the fitting portion 351B and the range of motion of the relay member 353 about the axis passing through the fitting portion 353A are each the range of 90 degrees, for example.

The relay member 353 includes separate members, namely, a member 353-1 on the distal end side and a member 353-2 on the rear end side. As indicated by the ellipse #3, the relay member 353 is rotatable about the central axis of the circular cross section at a coupling portion 353B between the member 353-1 and the member 353-2. The other movable portions also have a basically similar range of motion.

In such a way, the attaching/detaching member 351 having the attaching/detaching portion 351A at the distal end, the relay member 353 for connecting the attaching/detaching member 351 and the base member 355 to each other, and the base member 355 having the rear end to which the attaching/detaching portion 356 is connected are rotatably connected to one another through the hinge portions. The movement of each movable portion is controlled by a controller in the cooking robot 302 according to an instruction command.

FIG. 32 is a diagram illustrating exemplary connection between the cooking arms and the controller.

As illustrated in FIG. 32, the cooking arms 321 and a controller 361 are connected to each other through wires in a space 311B formed inside the housing 311. In the example of FIG. 32, the cooking arms 321-1 to 321-4 and the controller 361 are connected to each other through respective wires 362-1 to 362-4. The wires 362-1 to 362-4 having flexibility are appropriately bent depending on the positions of the cooking arms 321-1 to 321-4.

<Configuration of Cooking Robot 302>

FIG. 33 is a block diagram illustrating a configuration example of the cooking robot 302.

The cooking robot 302 includes the controller 361 (FIG. 32) serving as a control device configured to control the operation of the cooking robot 302 and each unit connected thereto. Of the components illustrated in FIG. 33, the components that are the same as the components described above are denoted by the same reference signs. The redundant description is appropriately omitted.

To the controller 361, other than the cooking arms 321, a camera 401, a sensor 402, and a communication unit 403 are connected.

The controller 361 includes a computer including a CPU, a ROM, a RAM, a flash memory, and the like. The controller 361 executes, by the CPU, a predetermined program to control the operation of the entire cooking robot 302. The data processing device 301 may include the controller 361.

For example, the controller 361 controls the communication unit 403 to transmit an image taken by the camera 401 and sensor data measured by the sensor 402 to the data processing device 301.

The controller 361 realizes an instruction command acquiring unit 411 and an arm control unit 412 by executing a predetermined program.

The instruction command acquiring unit 411 acquires an instruction command transmitted from the data processing device 301 and received by the communication unit 403. The instruction command acquired by the instruction command acquiring unit 411 is supplied to the arm control unit 412.

The arm control unit 412 controls the operation of the cooking arm 321 according to an instruction command acquired by the instruction command acquiring unit 411.

The camera 401 captures the cooking robot 302 and its surroundings and outputs the captured image to the controller 361. The camera 401 is provided at various positions, i.e., in front of the cooking assistance system 312 or at the distal end of the cooking arm 321, for example.

The sensor 402 includes various sensors such as a temperature and humidity sensor, a pressure sensor, an optical sensor, a distance sensor, a motion sensor, a positioning sensor, and a vibration sensor. The sensor 402 performs measurement at a predetermined cycle. Sensor data indicating the result of the measurement by the sensor 402 is supplied to the controller 361.

The camera 401 and the sensor 402 may be provided at positions distant from the housing 311 of the cooking robot 302.

The communication unit 403 is a wireless communication module such as a wireless LAN module or a mobile communication module supporting LTE (Long Term Evolution). The communication unit 403 communicates with the data processing device 301 and an external device such as the server on the Internet.

As illustrated in FIG. 33, the cooking arm 321 includes a motor 421 and a sensor 422.

The motor 421 is provided at each joint portion of the cooking arm 321. The motor 421 rotates about the axis under the control of the arm control unit 412. An encoder configured to measure the amount of rotation of the motor 421, a driver configured to adaptively control the rotation of the motor 421 according to the result of measurement by the encoder, and the like are also provided at each joint portion.

The sensor 422 includes, for example, a gyro sensor, an acceleration sensor, or a touch sensor. While the cooking arm 321 is operating, the sensor 422 measures the angular velocity, acceleration, or the like of each joint portion and outputs information indicating the measurement result to the controller 361. Sensor data indicating the result of measurement by the sensor 422 is also appropriately transmitted from the cooking robot 302 to the data processing device 301.

<Configuration of Data Processing Device 301>

FIG. 34 is a block diagram illustrating a functional configuration example of the data processing device 301.

At least some of the functional units illustrated in FIG. 34 are realized by the CPU of the computer of the data processing device 301 executing a predetermined program. The data processing device 301 has a configuration basically similar to the configuration of the cooking simulation device 1 described with reference to FIG. 16.

As illustrated in FIG. 34, the data processing device 301 realizes a command generating unit 431. The command generating unit 431 includes a recipe data acquiring unit 451, a robot state estimating unit 452, a control unit 453, and a command output unit 454.

The recipe data acquiring unit 451 acquires recipe data newly generated by the cooking simulation device 1 or the like and outputs the recipe data to the control unit 453.

The robot state estimating unit 452 receives image and sensor data transmitted from the cooking robot 302. From the cooking robot 302, an image captured by the camera of the cooking robot 302 and sensor data measured by the sensor provided at a predetermined position of the cooking robot 302 are transmitted at a predetermined cycle. In the image captured by the camera of the cooking robot 302, the cooking robot 302 and its surroundings appear.

The robot state estimating unit 452 analyzes image and sensor data transmitted from the cooking robot 302 and estimates the cooking robot 302 and its surroundings and the progress of a cooking process such as the states of the cooking arms 321 and the states of ingredients. Information indicating the cooking robot 302 and its surroundings and the like estimated by the robot state estimating unit 452 is supplied to the control unit 453.

The control unit 453 generates, in reference to a cooking process data set described in recipe data supplied from the recipe data acquiring unit 451, an instruction command for controlling the cooking robot 302. For example, an instruction command for causing the cooking arms 321 to perform an operation based on cooking operation information included in a cooking process data set is generated.

An instruction command is generated also with reference to the cooking robot 302 and its surroundings and the like estimated by the robot state estimating unit 452. The instruction command generated by the control unit 453 is supplied to the command output unit 454.

The command output unit 454 transmits an instruction command generated by the control unit 453 to the cooking robot 302.

<Configuration of Cooking Simulation Device 1>

FIG. 35 is a block diagram illustrating a functional configuration example of the cooking simulation device 1.

Of the components illustrated in FIG. 35, the components that are the same as the components described above are denoted by the same reference signs. The redundant description is appropriately omitted. As illustrated in FIG. 35, the cooking simulation device 1 realizes, in addition to the simulation calculation unit 132 and the presentation unit 133, an original recipe data acquiring unit 501 and a NEW recipe data generating unit 502.

The original recipe data acquiring unit 501 communicates with the server configured to manage recipe data, for example, to acquire original recipe data from which NEW recipe data is generated. The original recipe data acquired by the original recipe data acquiring unit 501 is supplied to the simulation calculation unit 132 and the NEW recipe data generating unit 502.

The simulation calculation unit 132 selects a reference ingredient from ingredients described in original recipe data and simulates the affinity of the reference ingredient and another ingredient in combination.

The presentation unit 133 presents the result of simulation by the simulation calculation unit 132 to the chef.

The NEW recipe data generating unit 502 receives selection of an ingredient by the chef who has checked a simulation result presented by the presentation unit 133. In ingredient selection, a predetermined ingredient is selected from appropriate ingredients by using voice. The NEW recipe data generating unit 502 updates the content of the original recipe data such that the ingredient selected by the chef is used in the cooking processes, to thereby generate NEW recipe data.

<Operation of Cooking Simulation Device 1>

With reference to the flowchart of FIG. 36, NEW recipe data generation processing by the cooking simulation device 1 is described. The processing of FIG. 36 starts when, for example, original recipe data is selected by the chef.

In Step S101, the original recipe data acquiring unit 501 communicates with the server configured to manage recipe data and acquires original recipe data.

In Step S102, the simulation calculation unit 132 analyzes the content described in the original recipe data and detects ingredients supposed to be used in the cooking processes.

In Step S103, the simulation calculation unit 132 selects a reference ingredient from the ingredients supposed to be used in the cooking processes and simulates the affinity of the reference ingredient and another ingredient in combination.

In Step S104, the presentation unit 133 presents the result of the simulation by the simulation calculation unit 132 to the chef.

In Step S105, the NEW recipe data generating unit 502 receives selection of an ingredient by the chef who has checked the simulation result presented by the presentation unit 133.

In Step S106, the NEW recipe data generating unit 502 updates the content of the original recipe data such that the ingredient selected by the chef is used in the cooking processes and thereby generates NEW recipe data.

<Operation of Data Processing Device 301>

With reference to the flowchart of FIG. 37, processing by the data processing device 301 configured to control the operation of the cooking robot 302 is described.

In the processing illustrated in FIG. 37, NEW recipe data generated by the cooking simulation device 1 is acquired, and the cooking robot 302 is controlled according to the description of the NEW recipe data.

In Step S111, the recipe data acquiring unit 451 (FIG. 34) acquires NEW recipe data generated by the cooking simulation device 1 via, for example, the server configured to manage recipe data.

In Step S112, the control unit 453 selects a predetermined cooking operation according to the cooking process data sets described in the recipe data and generates an instruction command for causing the cooking robot 302 to perform the selected cooking operation. For example, the cooking process data sets are selected in order of cooking processes, and the cooking operations included in the selected cooking process are selected in order of execution.

In Step S113, the command output unit 454 transmits the instruction command to the cooking robot 302 and causes the cooking robot 302 to execute the cooking operation.

In Step S114, the robot state estimating unit 452 estimates the state of the cooking robot 302.

In Step S115, the control unit 453 determines whether or not all the cooking operations have ended. In a case where it is determined in Step S115 that all the cooking operations have not yet ended, the processing returns to Step S112 where the next cooking operation is selected, and the above-mentioned processing is repeated.

In a case where it is determined in Step S115 that all the cooking operations have ended, the processing ends. At this time, the dish is complete according to the NEW recipe data generated by the cooking simulation device 1.

In such a way, the cooking simulation device 1 generates recipe data for controlling the robot configured to cook using the cooking arms.

FIG. 38 is a diagram illustrating an exemplary system configuration.

An information processing unit 511 illustrated in FIG. 38 includes the NEW recipe data generating unit 502 and the command generating unit 431. The configuration of the command generating unit 431 is the same as the configuration described with reference to FIG. 34.

The command generating unit 431 of FIG. 38 acquires NEW recipe data generated by the NEW recipe data generating unit 502 and controls the cooking robot 302 according to the NEW recipe data.

In such a way, the information processing unit 511 including the NEW recipe data generating unit 502 and the command generating unit 431 may be provided in the same device such as the cooking simulation device 1 or the data processing device 301.

<<3. Modified Example>>

<System Configuration>

FIG. 39 is a diagram illustrating a configuration example of an information processing system.

In the information processing system illustrated in FIG. 39, an information processing server 521 performs at least one of a cooking-related simulation and NEW recipe data generation. The information processing server 521 has a configuration similar to the configuration of the cooking simulation device 1 described with reference to FIG. 17, FIG. 22, and FIG. 35.

For example, in a case where the information processing server 521 has the function of performing a cooking-related simulation, the information processing server 521 and the cooking simulation device 1 provided on the chef side communicate with each other via the Internet. Data regarding the voice of the chef or the like is transmitted from the cooking simulation device 1 to the information processing server 521.

The simulation calculation unit 132 of the information processing server 521 performs a cooking-related simulation in response to the request from the chef and transmits information indicating the simulation result to the cooking simulation device 1, and the cooking simulation device 1 presents the information to the chef.

In such a way, the information processing server 521 on the Internet can perform a cooking-related simulation.

Although the cooking robot 302 is controlled in the control system of FIG. 24, electronic cooking equipment such as a microwave oven may be controlled according to recipe data. The electronic cooking equipment performs a cooking operation according to an instruction command supplied from the data processing device 301, to cook.

In such a way, recipe data can be used for controlling various apparatuses configured to automatically perform cooking operations.

Although the cooking simulation device 1 is used by the chef in the above, the cooking simulation device 1 may be used by an ordinary person at home.

<Other Examples>

•Configuration Example of Computer

The series of processing processes described above can be executed by hardware or software. In a case where the series of processing processes is executed by software, a program configuring the software is installed on a computer incorporated in dedicated hardware or a general-purpose personal computer.

A program to be installed is provided by being recorded on the removable medium 114 that is illustrated in FIG. 16 and includes an optical disc (CD-ROM (Compact Disc-Read Only Memory), DVD (Digital Versatile Disc), or the like), a semiconductor memory, or the like. Further, the program may be provided through a wired or wireless transmission medium such as a local area network, the Internet, or digital broadcast. The program can be installed in the ROM 102 or the storage unit 111 in advance.

As for the program that is executed by the computer, the processing processes of the program may be performed chronologically in the order described herein or in parallel. Alternatively, the processing processes may be performed at appropriate timings, for example, when the program is called.

Note that, herein, "system" means an aggregation of multiple components (devices, modules (parts), or the like), and it does not matter whether all the components are in the same housing or not. Hence, multiple devices that are accommodated in separate housings and connected to each other via a network and a single device including multiple modules accommodated in a single housing are both "system."

The effects described herein are merely exemplary and not limitative, and other effects may be provided.

The embodiment of the present technology is not limited to the embodiment described above, and various modifications can be made without departing from the gist of the present technology.

For example, the present technology can be configured as cloud computing in which a single function is shared and processed by multiple devices via a network.

Further, the steps of the flowcharts described above can be executed by a single device or shared and executed by multiple devices.

Furthermore, in a case where multiple processing processes are included in a single step, the multiple processing processes included in the single step can be executed by a single device or shared and executed by multiple devices.

[Reference Signs List]

| | |
|---|---|
| 1: | Cooking simulation device |
| 11: | Ingredient affinity information DB |
| 21: | Flavor subjective information DB |
| 22: | Sensing information DB |
| 23: | Chemical structure information DB |
| 131: | Simulation subject recognizing unit |
| 132: | Simulation calculation unit |
| 133: | Presentation unit |
| 161: | Sensing information generating unit |
| 162: | Flavor subjective information generating unit |
| 163: | Ingredient affinity information generating unit |
| 501: | Original recipe data acquiring unit |
| 502: | NEW recipe data generating unit |

**Claims**

1. An information processing device comprising:
   a presentation unit configured to present, using sensor information obtained by measuring, by a sensor, a flavor of an ingredient that is used in cooking, ingredient affinity information indicating an affinity of the ingredients in combination, and flavor subjective information indicating a subjective evaluation by people regarding the flavor of the ingredient or a flavor of the ingredients in combination, a state of the affinity of the ingredients in combination.

2. The information processing device according to claim 1, wherein the presentation unit presents the state of the affinity of the ingredients in combination in conjunction with the flavors of the ingredients described in a recipe or the flavor of the ingredients in combination.

3. The information processing device according to claim 2, wherein the presentation unit presents the state of the affinity of the ingredients in a new combination that is not described in the recipe.

4. The information processing device according to claim 1, wherein the presentation unit presents the state of the affinity of the ingredients in combination in conjunction with an action of a user cooking.

5. The information processing device according to claim 4, wherein the presentation unit presents the state of the affinity of the ingredients in a new combination that the user cooking has not recognized.

6. The information processing device according to claim 1, wherein the presentation unit displays a first icon representing a first ingredient and a second icon representing a second ingredient that achieves an affinity satisfying a predetermined condition in combination with the first ingredient, on a display screen side by side.

7. The information processing device according to claim 6, wherein the presentation unit regards the first icon as a reference and displays the second icon in plural number around the first icon.

8. The information processing device according to claim 6, wherein the presentation unit displays, in a case where the first ingredient and the second ingredient achieve the affinity satisfying the condition in combination, the first icon and the second icon being merged on the display screen in a manner that the affinity of the first ingredient and the second ingredient in combination is identifiable.

9. The information processing device according to claim 6, wherein the presentation unit displays, in a case where the first ingredient and the second ingredient achieve the affinity satisfying the condition in combination, the first icon and the second icon being merged on the display screen in a manner that a flavor of the first ingredient and the second ingredient in combination is identifiable.

**10.** The information processing device according to claim 6, wherein the presentation unit moves at least one of the first icon and the second icon depending on the affinity of the first ingredient and the second ingredient in combination.

**11.** The information processing device according to claim 1, wherein the presentation unit presents the affinity of the ingredients in combination by using chemical structure information indicating chemical structures of the ingredients.

**12.** The information processing device according to claim 1, wherein the sensor information includes information obtained by measuring, by a flavor measuring instrument, the flavor of the ingredient in a cooking process.

**13.** The information processing device according to claim 1, wherein the flavor subjective information includes information indicating a flavor subjective evaluation value provided by people who have eaten the ingredient.

**14.** The information processing device according to claim 1, further comprising:
a recipe generating unit configured to generate a new recipe by using a combination of the ingredients selected by a user from combinations of the ingredients presented.

**15.** The information processing device according to claim 14, further comprising:
a command generating unit configured to generate, according to recipe data indicating the new recipe generated by the recipe generating unit, an instruction command for causing a cooking robot to execute a cooking operation corresponding to each process described in the new recipe.

**16.** An information processing method comprising:
causing an information processing device to present, with use of sensor information obtained by measuring, by a sensor, a flavor of an ingredient that is used in cooking, ingredient affinity information indicating an affinity of the ingredients in combination, and flavor subjective information indicating a subjective evaluation by people regarding the flavor of the ingredient or a flavor of the ingredients in combination, a state of the affinity of the ingredients in combination.

FIG.1

EP 4 089 621 A1

**F I G . 2**

# FIG.3

EP 4 089 621 A1

FIG. 4

P1

Pinot-Noir

EP 4 089 621 A1

# FIG.5

FIG.6

# FIG.7

# FIG.8

# FIG.9

CHEF HAS PUT POTATO IN POT

SIMULATE STATE OF INGREDIENT

EP 4 089 621 A1

EP 4 089 621 A1

# FIG.10

INGREDIENT AFFINITY
INFORMATION DB

11

INGREDIENT AFFINITY INFORMATION

| | INGREDIENT A | INGREDIENT B | INGREDIENT C | . . . |
|---|---|---|---|---|
| INGREDIENT A | | | | |
| INGREDIENT B | | | | |
| INGREDIENT C | | | | |
| ⋮ | | | | |

FIG.11

flavor = taste + aroma + texture

# FIG.12

FLAVOR SUBJECTIVE INFORMATION DB — 21

SENSING INFORMATION DB — 22

CHEMICAL STRUCTURE INFORMATION DB — 23

EP 4 089 621 A1

# FIG.13

DB OF SUBJECTIVE EVALUATIONS RELATED
TO FLAVORS OF RESPECTIVE INGREDIENTS

21 ~ FLAVOR SUBJECTIVE
INFORMATION DB

FLAVOR SUBJECTIVE EVALUATION IS REPRESENTED BY (e-DIMENSIONAL)
VECTOR REPRESENTATION IN FLAVOR SUBJECTIVE EVALUATION SPACE E

E (INGREDIENT A) = [EA1, EA2, ···, EAi]
E (INGREDIENT B) = [EB1, EB2, ···, EBi]

.
.
.
.

EP 4 089 621 A1

# FIG.14

DB OF SENSING RESULTS OF FLAVORS
OF RESPECTIVE INGREDIENTS

22 — SENSING
INFORMATION DB

SENSING INFORMATION IS REPRESENTED BY (s-DIMENSIONAL)
VECTOR REPRESENTATION IN SENSING INFORMATION SPACE S

S (INGREDIENT A) = [SA1, SA2, ···, SAi]
S (INGREDIENT B) = [SB1, SB2, ···, SBi]

.
.
.

EP 4 089 621 A1

# FIG.15

DB OF CHEMICAL STRUCTURES
OF RESPECTIVE INGREDIENTS

23 — CHEMICAL STRUCTURE
INFORMATION DB

CHEMICAL STRUCTURE INFORMATION IS REPRESENTED BY (c-DIMENSIONAL)
VECTOR REPRESENTATION IN CHEMICAL STRUCTURE SPACE Ch

Ch (INGREDIENT A) = [NA1, NA2, ⋯, NAi]
Ch (INGREDIENT B) = [NB1, NB2, ⋯, NBi]

# FIG.16

EP 4 089 621 A1

```
            101           102           103
             |             |             |
         ┌───────┐     ┌───────┐     ┌───────┐
         │  CPU  │     │  ROM  │     │  RAM  │
         └───────┘     └───────┘     └───────┘
             ↕             ↓             ↕        104
    ◁═══════════════════════════════════════════════▷
                            ↕

┌─────────────────────────────────────────────────────────────┐
│                  INPUT-OUTPUT INTERFACE                       │  ~105
└─────────────────────────────────────────────────────────────┘
    ↑        ↑         ↑         ↓        ↓        ↕         ↕         ↕
┌────────┐┌────────┐┌─────────┐┌───────┐┌───────┐┌────────┐┌─────────────┐┌───────┐
│MICRO-  ││ CAMERA ││OPERATION││DISPLAY││SPEAKER││STORAGE ││COMMUNICATION││ DRIVE │ ~113
│PHONE   ││        ││ UNIT    ││       ││       ││ UNIT   ││ UNIT        ││       │
└────────┘└────────┘└─────────┘└───────┘└───────┘└────────┘└─────────────┘└───────┘
   106       107        108       109      110      111        112           ↕
                                                                         ┌─────────┐
                                                                         │REMOVABLE│ ~114
                                                                         │ MEDIUM  │
                                                                         └─────────┘
```

1

## FIG.17

SIMULATION SUBJECT RECOGNIZING UNIT **131** → SIMULATION CALCULATION UNIT **132** → PRESENTATION UNIT **133**

# FIG.18

```
         ┌─────────────────────────────┐
         │      START COOKING          │
         │  SIMULATION PROCESSING      │
         └─────────────────────────────┘
                      │
                      ▼
         ┌─────────────────────────────┐ S1
         │     DETECT VOICE OF CHEF    │
         └─────────────────────────────┘
                      │
                      ▼
         ┌─────────────────────────────┐ S2
         │         CAPTURE CHEF        │
         └─────────────────────────────┘
                      │
                      ▼
         ┌─────────────────────────────┐ S3
         │  RECOGNIZE SIMULATION SUBJECT│
         └─────────────────────────────┘
                      │
                      ▼
         ┌─────────────────────────────┐ S4
         │      PERFORM SIMULATION     │
         └─────────────────────────────┘
                      │
                      ▼
         ┌─────────────────────────────┐ S5
         │   PRESENT SIMULATION RESULT │
         └─────────────────────────────┘
                      │
                      └──────────────────┘
```

FIG.19

EP 4 089 621 A1

# FIG.20

EP 4 089 621 A1

# FIG.21

# FIG.22

161 — SENSING INFORMATION GENERATING UNIT

141 — AROMA SENSOR

142 — TASTE SENSOR

143 — TEXTURE SENSOR

163 — INGREDIENT AFFINITY INFORMATION GENERATING UNIT

162 — FLAVOR SUBJECTIVE INFORMATION GENERATING UNIT

EP 4 089 621 A1

# FIG.23

DISH X RECIPE DATA

A1

1

A2

SIMULATE AFFINITY OF COMBINATION

USE SALMON

1

A3

UPDATE RECIPE DATA

1

A4

DISH X RECIPE DATA (NEW)

# FIG.24

DISH X
RECIPE DATA

→ A1

301
DATA PROCESSING
DEVICE

INSTRUCTION
COMMAND
→ A2

302

EP 4 089 621 A1

# FIG.25

RECIPE DATA

COOKING PROCESS DATA SET
(COOKING PROCESS #1)

COOKING PROCESS DATA SET
(COOKING PROCESS #2)

COOKING PROCESS DATA SET
(COOKING PROCESS #N)

# FIG.26

DISH X RECIPE DATA

COOKING PROCESS #1

COOKING PROCESS #2

COOKING PROCESS #N

302

# FIG.27

A

301

DATA PROCESSING
DEVICE

302

B

301

302

EP 4 089 621 A1

F I G . 2 8

321-1  321-2  321-3  321-4

312

311A

311

302

FIG.29

F I G . 3 0

FIG.31

FIG.32

# F I G . 3 3

F I G . 3 4

431 COMMAND GENERATING UNIT

452 ROBOT STATE ESTIMATING UNIT

451 RECIPE DATA ACQUIRING UNIT

453 CONTROL UNIT

454 COMMAND OUTPUT UNIT

# FIG.35

| ORIGINAL RECIPE DATA ACQUIRING UNIT | | SIMULATION CALCULATION UNIT | | PRESENTATION UNIT | | NEW RECIPE DATA GENERATING UNIT |
| --- | --- | --- | --- | --- | --- | --- |
| 501 | | 132 | | 133 | | 502 |

EP 4 089 621 A1

# FIG.36

```
┌─────────────────────────┐
│   START NEW RECIPE      │
│  GENERATION PROCESSING  │
└─────────────────────────┘
            │
            ▼
┌─────────────────────────────┐ S101
│ ACQUIRE ORIGINAL RECIPE DATA│
└─────────────────────────────┘
            │
            ▼
┌─────────────────────────────┐ S102
│   ANALYZE RECIPE DATA TO    │
│   DETECT USED INGREDIENT    │
└─────────────────────────────┘
            │
            ▼
┌─────────────────────────────┐ S103
│ SIMULATE AFFINITY OF INGREDIENT │
└─────────────────────────────┘
            │
            ▼
┌─────────────────────────────┐ S104
│   PRESENT SIMULATION RESULT │
└─────────────────────────────┘
            │
            ▼
┌─────────────────────────────┐ S105
│     RECEIVE SELECTION OF    │
│     INGREDIENT  BY CHEF     │
└─────────────────────────────┘
            │
            ▼
┌─────────────────────────────┐ S106
│ UPDATE RECIPE DATA SUCH THAT│
│ SELECTED  INGREDIENT IS USED│
└─────────────────────────────┘
            │
            ▼
        ┌───────┐
        │  END  │
        └───────┘
```

# FIG.37

```
      ╭─────────────────────────────╮
      │  START CONTROL PROCESSING   │
      │  OF DATA PROCESSING DEVICE  │
      ╰─────────────────────────────╯
                     │
                     ▼
      ┌─────────────────────────────┐
      │   ACQUIRE NEW RECIPE DATA    │  S111
      └─────────────────────────────┘
                     │
                     ▼
      ┌─────────────────────────────┐
      │  SELECT COOKING OPERATION AND │  S112
      │  GENERATE INSTRUCTION COMMAND │
      └─────────────────────────────┘
                     │
                     ▼
      ┌──────────────────────────────────────────────┐
      │ CAUSE COOKING ROBOT TO EXECUTE COOKING OPERATION │  S113
      └──────────────────────────────────────────────┘
                     │
                     ▼
      ┌─────────────────────────────┐
      │ ESTIMATE STATE OF COOKING ROBOT │  S114
      └─────────────────────────────┘
                     │
                     ▼
  NO ◁─────────────────────────────────────▷  S115
     │  HAVE ALL COOKING OPERATIONS ENDED?  │
                     │ YES
                     ▼
              ╭───────────╮
              │    END    │
              ╰───────────╯
```

# F I G . 3 8

INFORMATION PROCESSING UNIT 511

COMMAND GENERATING UNIT 431

NEW RECIPE GENERATING UNIT 502 → RECIPE DATA ACQUIRING UNIT 451 → CONTROL UNIT 453 → COMMAND OUTPUT UNIT 454

ROBOT STATE ESTIMATING UNIT 452 → CONTROL UNIT 453

EP 4 089 621 A1

FIG.39

EP 4 089 621 A1

<div style="text-align:center">**INTERNATIONAL SEARCH REPORT**</div>

| International application No. |
| --- |
| PCT/JP2020/048731 |

**A. CLASSIFICATION OF SUBJECT MATTER**
G06Q 50/12(2012.01)i; G01N 33/02(2006.01)i
FI: G06Q50/12; G01N33/02

According to International Patent Classification (IPC) or to both national classification and IPC

**B. FIELDS SEARCHED**

Minimum documentation searched (classification system followed by classification symbols)
G06Q50/12; G01N33/02

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2012-212414 A (NATIONAL UNIVERSITY CORPORATION KITAMI INSTITUTE OF TECHNOLOGY) 01 November 2012 (2012-11-01) entire text, all drawings | 1-16 |
| A | 田中 真, 追加食材と参考レシピの推薦によるオリジナル料理レシピ創出支援システム, 第5回データ工学と情報マネジメントに関するフォーラム（第11回日本データベース学会年次大会） [online], 31 May 2013, entire text, all drawings, non-official translation (TANAKA, Makoto, "Recipe creation support system of original cooking by recommending additional ingredients and reference recipes", The 5th forum on data engineering and information management (The 11th annual conference of the Database Society of Japan)) | 1-16 |

☐ Further documents are listed in the continuation of Box C.  ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 18 March 2021 (18.03.2021) | 30 March 2021 (30.03.2021) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

| International application No. |
| --- |
| PCT/JP2020/048731 |

| Patent Documents referred in the Report | Publication Date | Patent Family | Publication Date |
| --- | --- | --- | --- |
| JP 2012-212414 A | 01 Nov. 2012 | (Family: none) | |

Form PCT/ISA/210 (patent family annex) (January 2015)

**REFERENCES CITED IN THE DESCRIPTION**

**Patent documents cited in the description**

- JP 2019020912 A

- WO 2017085777 A